# EUROPEAN PATENT SPECIFICATION

(11) **EP 1 092 720 B1**
(45) Date of publication and mention of the grant of the patent: **12.01.2005**
(21) Application number: 00308915.8
(22) Date of filing: 10.10.2000
(51) Int. Cl.: C07D 487/04, C07D 401/12, C07D 401/14

(54) **Process for the preparation of pyrazolo [4,3-d] pyrimidin-7-ones-3-pyridylsulphonyl compounds and intermediates thereof**
Verfahren zur Herstellung von 3-pyridylsulphonylpyrazolo[4,3-d]pyrimidin-7-one und Zwischenprodukte
Procédé pour la préparation de 3-pyridylsulphonylpyrazolo[4,3-d]pyrimidin-7-ones et intermédiaires

(30) Priority: 11.10.1999 GB 9924042; 28.07.2000 GB 0018667
(43) Date of publication of application: 18.04.2001
(73) Proprietor: Pfizer Limited, Sandwich, Kent CT13 9NJ (GB); PFIZER INC., New York, N.Y. 10017 (US)
(72) Inventor: Devries, Keith Michael, Groton, Connecticut 06340 (US); Levett, Philip Charles, Sandwich, Kent CT13 9NJ (GB); Negri, Joanna Teresa, Groton, Connecticut 06340 (US); Wood, Albert Shaw, Sandwich, Kent CT13 9NJ (GB)
(74) Representative: Atkinson, Jonathan David Mark

(56) References cited:
- EP-A- 0 995 750
- WO-A-98/49166
- WO-A-99/54333

## Description

This invention relates to a series of pyrazolo[4,3-d]pyrimidin-7-ones-3-pyridylsulphonyl compounds of formula I (as defined below) and intermediates thereof. More notably, most of the compounds of interest are inhibitors of type 5 cyclic guanosine 3',5'-monophosphate phosphodiesterase (cGMP PDE5) and have utility in a variety of therapeutic areas ( such as male erectile dysfunction). Particular compounds of interest are 1-ethyl-4-{5-[3-ethyl-6,7-dihydro-7-oxo-2-(2-pyridylmethyl)-2H-pyrazolo[4,3-d]pyrimidin-5-yl]-6-(2-methoxyethoxy)-3-pyridylsulfonyl}piperazine (hereinafter compound of formula IA) and ( *R* )-1-ethyl-4-[5-(3-ethyl-6,7-dihydro-2-methyl-7-oxo-2H-pyrazolo[4,3-d]pyrimidin-5-yl)-6-(2-methoxy-1-methylethoxy)-5-pyridylsulphonyl]piperazine (hereinafter the compound of formula IB). An alternative name for (IB) is (+)-3-Ethyl-5-[5-(4-ethylpiperazin-1-ylsulphonyl)-2-(2-methoxy-1( R )-methyl ethoxy)pyridin-3-yl]-2-methyl-2,6-dihydro-7H-pyrazolo[4,3-d]pyrimidin-7-one.

Processes for the preparation of many of the compounds of formula I are disclosed in WO98/49166 and PCT/IB99/00519 (published as WO99/54333). In particular, examples 4 and 118 of PCT/ IB99/OO519 disclose a process for preparing compounds IA and IB. EP 0995750 discloses the use of 5-substituted pyrazolopyrimidin-7-ones for the treatment of sexuel dysfunction and a process for preparing these compounds. In the process, sulfonction of the cyclised pyrimidin-7-one followed by reaction with an amine leads to the target compounds. This document does not disclose any alkoxide exchange process and the required alkoxide group is introduced into the molecule at an early stage in the synthesis.

According to a first aspect of the invention there is provided a process for the preparation of a compound of formula (I) or a salt thereof 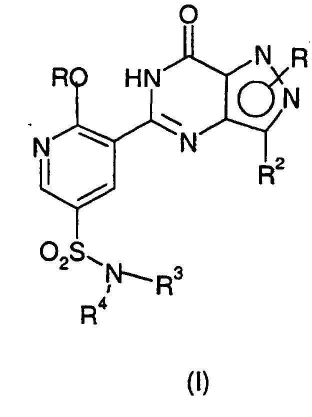 wherein
R is C₁ to C₆ alkyl optionally substituted with one or two substituents selected from C₃ to C₅ cycloalkyl, OH, C₁ to C₄ alkoxy, benzyloxy, NR⁵R⁶, phenyl, furanyl and pyridinyl; C₃ to C₆ cycloalkyl; 1-(C₁ to C₄ alkyl)piperidinyl; tetrahydrofuranyl or tetrahydropyranyl and wherein said C₁ to C₆ alkyl or said C₁ to C₄ alkoxy groups are optionally substituted by haloalkyl;
R¹ (which can be linked to either nitrogen of the pyrazole ring) is C₁ to C₃ alkyl, optionally substituted with phenyl, Het or a N linked heterocyclic group selected from piperidinyl and morpholinyl and wherein said phenyl group is optionally substituted by: C₁ to C₄ alkyl which is optionally substituted by haloalkyl or haloalkoxy; or C₁ to C₄ alkoxy; or halo or CN;
R² is C₁ to C₆ alkyl;
and Het is a C-linked 6-membered heterocyclic group containing one or two nitrogen atoms, optionally in the form of its mono-N-oxide, or a C-linked 5-membered heterocyclic group containing two or three nitrogen atoms, wherein either of said heterocyclic groups is optionally substituted with C₁ to C₄ alkyl or C₁ to C₄ alkoxy or NHR⁷wherein R⁷ is H, C₁ to C₄ alkyl or C₁ to C₄ alkanoyl.
R³ and R⁴, together with the nitrogen atom to which they are attached, form a 4-R⁸-piperazinyl group optionally substituted with one or two C₁ to C₄ alkyl groups and optionally in the form of its 4-N-oxide;
R⁵ and R⁶ are each independently selected from H and C₁ to C₄ alkyl optionally substituted with C₃ to C₅ cycloalkyl or C₁ to C₄ alkoxy, or, together with the nitrogen atom to which they are attached, form an azetidinyl, pyrrolidinyl, piperidinyl or morpholinyl group;
R⁸ is H; C₁ to C₄ alkyl optionally substituted with one or two substituents selected from OH, NR⁵R⁶, CONR⁵R⁶, phenyl optionally substituted with
C₁ to C₄ alkoxy, benzodioxolyl and benzodioxanyl; C₃ to C₆ alkenyl; pyridinyl or pyrimidinyl;
said process comprising reacting a compound of formula (II), (III) or (IV) in the presence of ⁻OR and a hydroxide trapping agent wherein the compounds of formula II are formed from the compounds of formula III or in the case of compounds of formula (IV) reacting in the presence of an auxillary base and a hydroxide trapping agent (i.e. ⁻OR is substituted by the auxiliary base) wherein X is a leaving group and R¹ to R⁴ are as defined above.

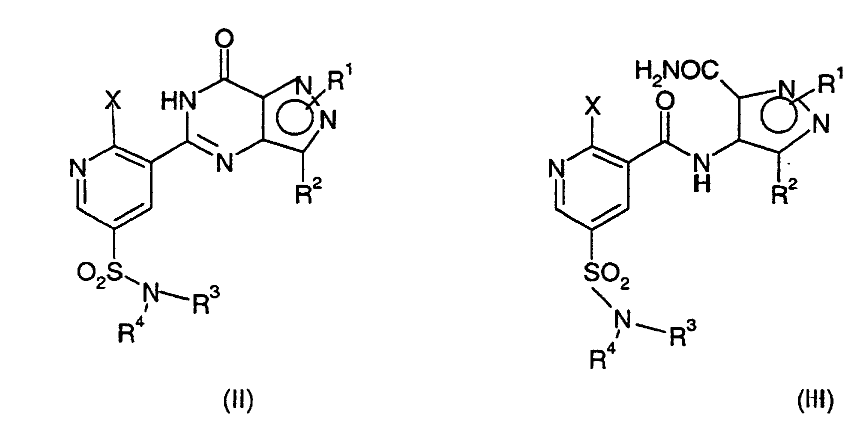

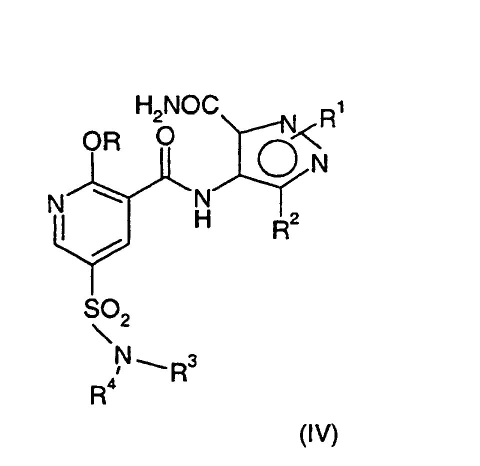

In the above definition, unless otherwise indicated, alkyl, alkoxy and alkenyl groups having three or more carbon atoms, and alkanoyl groups having four or more carbon atoms, may be straight chain or branched chain. The term halo atom includes, Cl, Br, F, and I. Haloalkyl and haloalkoxy include CF₃ and OCF₃ respectively.

The compounds of formulae (I) may contain one or more chiral centres and therefore can exist as stereoisomers, i.e. as enantiomers or diastereoisomers, as well as mixtures thereof. The invention relates to formation of both the individual stereoisomers of the compounds of formulae (I) and any mixture thereof.

In first and second preferred embodiments of the invention compounds of formulae (IA) and (IB) are prepared.

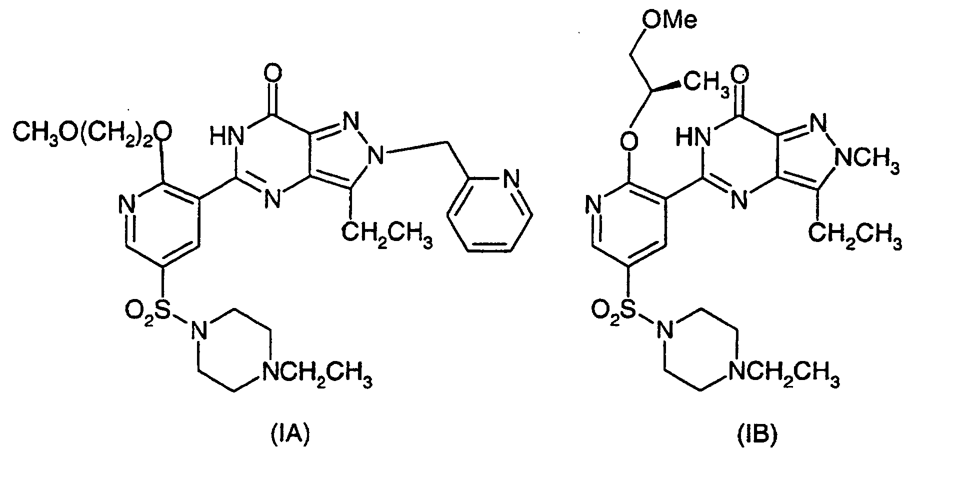

Accordingly, in a preferred aspect of the invention there is provided a process for the preparation of a compound of formula (IA) or (IB) 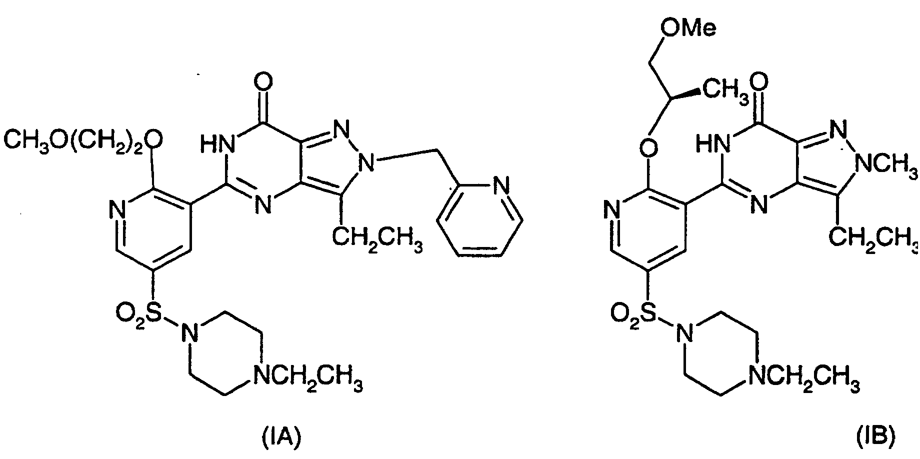 comprising reacting a compound of formula (IIA), (IIIA) or (IVA), and (IIB), (IIIB) or (IVB) respectively 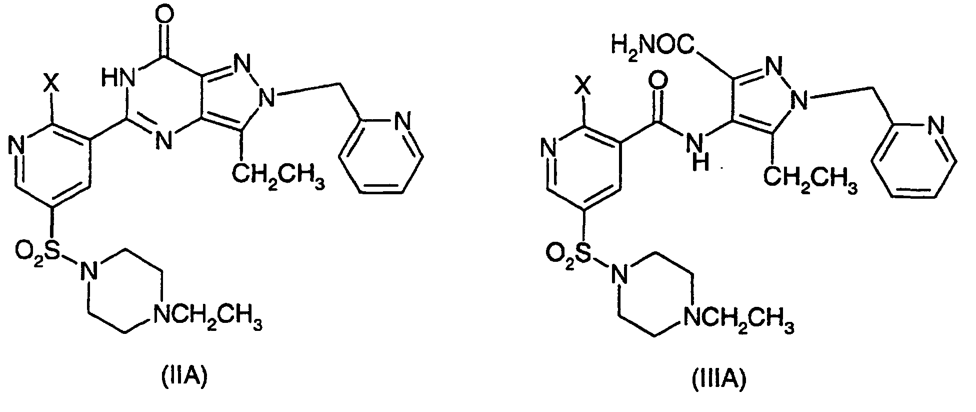 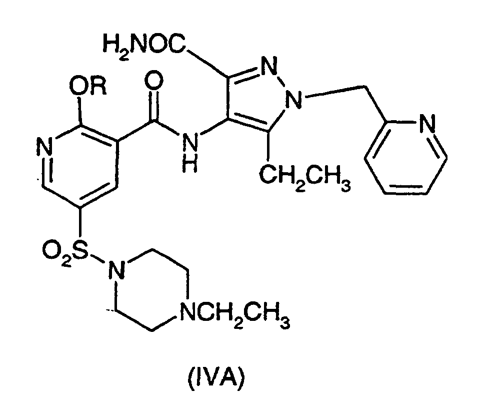 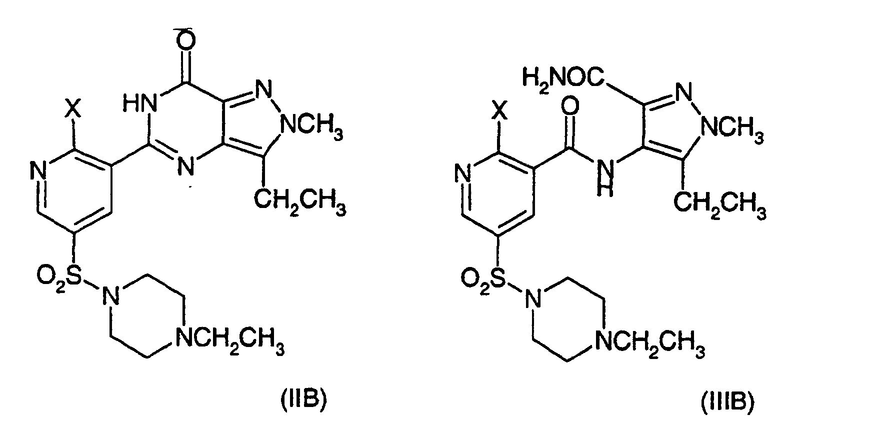 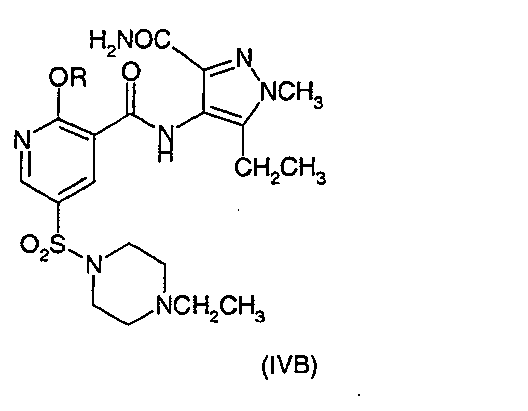 in the presence of ⁻OR and a hydroxide trapping agent, or alternatively in the case of compounds of formula (IVA) and (IVB) reacting in the presence of a hydroxide trapping agent and an auxiliary base wherein OR in the case of formation of compound (IA) is CH₃O(CH₂)₂O-, and OR in the case of formation of compound (IB) is *(R)*-CH₃OCH₂CH(CH₃)O- and wherein X in formulae (IIA) to (IIIA) and formulae (IIB) to (IIIB) is a leaving group.

Intermediates of the general formula (IV) and more specifically (IVA) and (IVB), where novel, form further aspects of the invention.

As a result of use of the hydroxide trapping agent, a particular advantage of the present process over that of the prior art (PCT/IB99/00519) is that a higher yield of final product (compounds of formula (I, IA, IB) and intermediate compounds (VI, VIA, VIB) can be obtained. In a preferred embodiment the compound of formula (I) are obtained in good yield without intermediate isolation.

It is most advantageous to form the compounds of formula (I) from intermediates of formula (III) since the cyclisation step (III to II) and the nucleophilic displacement of X by ⁻OR (II to I) can be carried out in a one pot reaction. Furthermore the process can be run at ambient pressure whereas the cyclisation step of the 2 step process requires higher pressures where XH is a lower alkanol e.g. methanol, ethanol, 1-propanol and 2-propanol. For example, the formation of compound (IIA) from (IIIA) in example 1B of PCT/IB99/00519 was under high pressure whereas in the present process (IA) can be formed from (IIIA) under ambient pressure.

In a further aspect of the invention, there is provided a process for the formation of compounds of formula (II) (more particularly IIA and IIB) comprising cyclising compounds of formula (III) (more particularly IIIA and IIIB) in the presence of said hydroxide trapping agent. Again, this step benefits from the higher yield and quality provided by using the hydroxide trapping agent.

Of course the trapping agent technology could be used to form compounds of formula (IV) (more particularly IVA and IVB) from compounds of formula (III) (more particularly IIIA and IIIB respectively) in the presence of ⁻OR, advantageously up to about 1 molar equivalent of ⁻OR (to compounds (III)). If substantially more than 1 equivalent of ⁻OR was used, the reaction would proceed through to compounds (I) (more particularly IA or IB).

Preferably the hydroxide trapping agent is an ester.

More preferably said hydroxide trapping agent is an ester of the formula:

TOC(O)W

wherein OT is OR or the residue of a bulky alcohol or a non-nucleophilic alcohol or TOH is an alcohol which can be azeotropically removed during the reaction;
and C(O)W is the residue of a carboxylic acid.

To further improve yields of final product and reduce impurities, preferably C(O)W is the residue of a sterically hindered carboxylic acid and/or a carboxylic acid which does not contain an enolisable proton (e.g. pivalic acid).

For example, where X is OEt in compound (IIA) and (IIIA) the ester trapping agent can be ethyl acetate (i.e. OT=X and C(O)W is a residue of acetic acid), more preferably ethyl pivalate (OT=X and C(O)W is the residue of pivalic acid- i.e. a carboxylic acid with no enolisable proton), 2-methoxyethylacetate (OT=OR and C(O)W is a residue of acetic acid), or most preferably 2-methoxyethylpivalate (OT=OR and C(O)W is a residue of a carboxylic acid with no enolisable proton).

Preferably X is selected from the group consisting of optionally substituted arylsulphonyloxy, preferably phenylsulphonyloxy, more preferably a para substituted aryl (phenyl) such as by a C₁-C₄ alkyl group e.g. p-toluenesulphonyloxy;
C₁-C₄ alkylsulphonyloxy e.g. methanesulphonyloxy;
nitro or halo substituted benzenesulphonyloxy preferably para substituted e.g. p-bromobenzenesulfonyloxy or p-nitrobenzenesulphonyloxy; C₁-C₄ perfluoroalkylsulphonyloxy e.g. trifluoromethylsulphonyloxy; optionally substituted aroyloxy such as benzoyloxy;
C₁-C₄ perfluoroalkanoyloxy such as trifluoroacetyloxy;
C₁-C₄ alkanoyloxy such as acetyloxy;
halo; diazonium; C₁-C₄ primary and secondary alkoxy such as methoxy; quatenaryammonium C₁-C₄ alkylsulphonyloxy; halosulphonyloxy e.g. fluorosulphonyloxy and other fluorinated leaving groups; and diarylsulphonylamino e.g. ditosyl (NTs₂).

Most preferably, for formation of compounds of formula (I) more particularly (IA) and (IB), X is a C₁₋₆ alkoxy (advantageously ethoxy) since this lends itself to simpler and cheaper formation of compounds - for example see Schemes 1, 2 and 3 hereinafter.

However an advantage of using labile leaving groups such as chloro or fluoro may be that an inert solvent could then be used rather than ROH (which will often be more expensive). Thus only a sufficient amount of ⁻OR (such as from ROH) as reactant would be required.

⁻OR can act both as a nucleophile (to displace the leaving group by nucleophilic substitution) and as a base (to bring about the cyclisation).

⁻OR can be generated in solution from, for example, a salt ZOR (wherein Z is a cation) such as a metal salt. More particularly an alkali (such as sodium or potassium) or alkaline earth metal salt of ⁻OR in a suitable solvent would give rise to ⁻OR in solution. For example, sodium 2-methoxyethoxide in a suitable solvent with intermediate (IIA) or (IIIA) would form compound (IA). In another embodiment, ⁻OR is formed *in situ* from ROH plus an auxiliary base (i.e. a base other than ⁻OR). However, in another system, ZOR could be used in the reaction system with an auxiliary base.

As will be appreciated the solvent in which the reaction takes place can be ROH or an inert solvent (or a mixture of both). By inert solvent we mean a solvent which will not form a nucleophile under the reaction conditions or if a nucleophile is formed it is sufficiently hindered or unreactive such that it does not substantially compete in the displacement reaction. When ROH is used as a source of ⁻OR, then a separate solvent is not essentially required but an (auxiliary) inert solvent (i.e. a solvent other than ROH) may be used as a co-solvent in the reaction.

Suitable solvents are as follows:
ROH, a secondary or tertiary C₄-C₁₂ alkanol, a C₃-C₁₂ cycloalkanol, a tertiary C₄-C₁₂ cycloalkanol, a secondary or tertiary (C₃-C₇ cycloalkyl)C₂-C₆ alkanol, a C₃-C₉ alkanone, 1,2-dimethoxyethane, 1,2-diethoxyethane, diglyme, tetrahydrofuran, 1,4-dioxan, toluene, xylene, chlorobenzene, 1,2-dichlorobenzene, acetonitrile, dimethyl sulphoxide, sulpholane, dimethylformamide, N-methylpyrrolidin-2-one, pyridine, and mixtures thereof.

More preferably, the solvent is ROH, a tertiary C₄-C₁₂ alkanol, a tertiary C₄-C₁₂ cycloalkanol, a tertiary (C₃-C₇ cycloalkyl)C₂-C₆ alkanol, a C₃-C₉ alkanone, 1,2-dimethoxyethane, 1,2-diethoxyethane, diglyme, tetrahydrofuran, 1,4-dioxan, toluene, xylene, chlorobenzene, 1,2-dichlorobenzene, acetonitrile, dimethyl sulphoxide, sulpholane, dimethylformamide, N-methylpyrrolidin-2-one, pyridine, and mixtures thereof.

Most preferably the solvent is ROH, which means that ⁻OR is formed *in situ* ,such as in the presence of an auxiliary base. For compounds (IA) and (IB) the solvent is preferably CH₃O(CH₂)₂OH and (*R*)-CH₃OCH₂CH(CH₃)OH respectively.

A wide range of auxiliary bases can be used in the process of the invention. Typically the bases would not substantially compete with ⁻OR in the nucleophilic substitution of X (i.e. they would be non nucleophilic) such as by being suitably sterically hindered.

Preferably the auxiliary base is selected from the group consisting of a sterically hindered base, a metal hydride, metal oxide, metal carbonate and metal bicarbonate.

The sterically hindered base is advantageously a metal salt of a sterically hindered alcohol or amine.

More preferably the auxiliary bases in accordance with the invention are selected from the group consisting of metal salts of a sterically hindered alcohol or amine such as a secondary or tertiary C₄-C₁₂ alkanol, a C₃-C₁₂ cycloalkanol and a secondary or teritary (C₃-C₈ cycloalkyl)C₁-C₆ alkanol, a N-(secondary or tertiary C₃-C₆ alkyl)-N-(primary, secondary or tertiary C₃-C₆ alkyl)amine, a N-(C₃-C₈ cycloalkyl)-N-(primary, secondary or tertiary C₃-C₆ alkyl)amine, a di(C₃-C₈ cycloalkyl)amine or hexamethyldisilazane; 1,5-diazabicyclo[4,3,0]non-5-ene and 1,8-diazabicyclo[5,4,0]undec-7-ene; a metal hydride, oxide, carbonate, and bicarbonate.

More preferably still, the auxiliary bases are selected from the group consisting of metal salts of a sterically hindered alcohol or amine such as a tertiary C₄-C₁₂ alkanol, a C₃-C₁₂ cycloalkanol and a tertiary (C₃-C₈ cycloalkyl)C₁-C₆ alkanol, a N-secondary or tertiary C₃-C₆ alkyl)-N-(primary, secondary or tertiary C₃-C₆ alkyl)amine, a N-(C₃-C₈ cycloalkyl)-N-(primary, secondary or tertiary C₃-C₆ alkyl)amine, a di(C₃-C₈ cycloalkyl)amine or hexamethyldisilazane; 1,5-diazabicyclo[4,3,0]non-5-ene and 1,8-diazabicyclo[5,4,0]undec-7-ene.

More preferably still, the auxiliary base is selected from the sterically hindered bases of the previous paragraph (i.e. all of the bases named except for the metal hydride, oxide, carbonate and bicarbonate).

Most preferably still, the auxiliary base is the metal salt of a tertiary C₄-C₆ alcohol such as the alkali or alkaline earth metal salts (e.g. Na/K) of t-butanol or t-amyl alcohol, or the base is potassium hexamethyldisilazone (KHMDS).

Most preferably, the auxiliary base is the alkali metal salt of t-butanol (e.g. potassium t-butoxide).

Preferably the metal of the salt of ZOR and the auxiliary base are independently selected from alkali metals (lithium, sodium, potassium, rubidium, caesium) or alkaline earth metals (beryllium, magnesium, calcium, strontium, barium). More preferably the metal is sodium or potassium.

To maximise yields, it is further preferred that at least about 1 molecular equivalent of auxiliary base and ⁻OR are used in accordance with the invention. If ⁻OR also functions as a base (i.e. there is no auxiliary base present) then preferably at least about 2 equivalents of ⁻OR are present. Suitably, at least about 1 equivalent of trapping agent (preferably at least about 2 equivalents) is present.

In a particularly preferred embodiment of the invention the compounds of formula (I) can surprisingly be provided of clinical quality material, thus obviating the need for subsequent purification steps.

The temperature of the reaction of compounds (III) and (IV) to (I) (such as the corresponding formation of compounds IA and IB) is preferably at least about 80°C, more preferably about 80 to about 130° C, more preferably still about 100 to about 130°C and most preferably about 115 to about 125°C. These temperatures are also applicable for the conversion of compounds (II) to (I), although the temperature in this case could also probably be lower (e.g. about 60° C) since there is no cyclisation taking place.

The reaction temperature attainable to effect the conversion of compounds of formulae (II) and (III) to compounds of formula (I) depends on the solvent, the nature of ⁻OR and X. When X is an alkoxy and ROH is the solvent, preferably XH (such as C₁₋₆ alkoxy) is removed azeotropically (of course the reaction vessel must be configured to distil over the azeotrope mixture) with ROH by running the reaction at the azeotrope temperature of XH and ROH. In this way the yield and quality of the final product can be further improved. For example, (where X is an alkoxy) the conversion of compound (IIA), (IIIA) or (IVA) to (IA) is preferably carried out at the azeotrope temperature of the alcohol (i.e. XH (preferably ethanol)) and 2-methoxyethanol.

Preferred embodiments of the invention are:
1. the synthesis of compound (IA) by reaction of compound (IIA) or (IIIA):
   a) with 2-methoxyethanol and auxiliary base, optionally in an inert solvent and in the presence of said trapping agent; or
   b) with ZO(CH₂)₂OCH₃ and an auxiliary base in 2-methoxyethanol or an inert solvent or both, in the presence of said trapping agent; or
   c) with ZO(CH₂)₂OCH₃ and 2-methoxyethanol or an inert solvent or both, in the presence of said trapping agent.

   Preferably, the trapping agent is CH₃O(CH₂)₂OC(O)W or CH₃OC(O)W wherein C(O)W is a residue of a carboxylic acid (preferably sterically hindered).
2. the synthesis of compound (IB) by reaction of compound (IIB) or (IIIB):
   a) with *(R)* -CH₃OCH₂CH(CH₃)OH and auxiliary base, optionally in an inert solvent and in the presence of said trapping agent; or
   b) with *(R)* -CH₃OCH₂CH(CH₃)OZ and an auxiliary base in *(R)* - CH₃OCH₂CH(CH₃)OH or an inert solvent or both, in the presence of said trapping agent; or)
   c) with *(R)* -CH₃OCH₂CH(CH₃)OZ and *(R)* -CH₃OCH₂CH(CH₃)OH or an inert solvent or both, in the presence of said trapping agent.

Preferably the trapping agent is (*R*)- CH₃OCH₂CH(CH₃)OC(O)W or CH₃OC(O)W wherein C(O)W is a residue of a carboxylic acid (preferably sterically hindered).

To maximise yields, it is further preferred that at least about 1 molecular equivalent of auxiliary base and ⁻OR are used in accordance with the invention. If ⁻OR also functions as a base (i.e. there is no auxiliary base present) then preferably at least about 2 equivalents of ⁻OR are present. Thus to maximise yields of compounds (IA) and (IB), suitably at least about 1 equivalent of trapping agent (preferably at least about 2 equivalents) is present. With respect to 1a) above, preferably there is at least about 2 molecular equivalents of base and at least about 1 molecular equivalent of trapping agent relative to the substrate (more preferably at least about 2.2 and 2.5 respectively). For 1b) above, preferably there is at least about 1 molecular equivalent of auxiliary base, trapping agent and ZO(CH₂)₂OCH₃ relative to the substrate (more preferably at least about 1.2 equivalents of auxiliary base and at least about 2.5 equivalents of trapping agent). For 1c) above, preferably there is at least about 2 molecular equivalents of ZO(CH₂)₂OCH₃ and at least about 1 equivalent of trapping agent relative to the substrate (more preferably at least about 2 and 2.5 equivalents respectively).

The compounds of general formula (III), (IIIA) and (IIIB) may be obtained from readily available starting materials for example, by the route depicted in the following reaction schemes. Reaction Scheme 1 is illustrated for compounds of general formula (I), Scheme 2 is for compound (IA) and Scheme 3 is illustrated for compound (IB).

With reference to Scheme 1, the intermediate of formula (VI) or a salt thereof is formed from a compound of formula (VIII), the exact process being dependent on leaving group X.

For compounds of formula (VI) or a salt thereof wherein X = arylsulfonyloxy, C₁-C₄ alkylsulfonyloxy, C₁-C₄ perfluoroalkylsulfonyloxy, aryloxy, C₁-C₄ perfluoroalkanoyloxy, C₁-C₄ alkanoyloxy, quarternaryammonium C₁-C₄ alkylsulfonyloxy or halosulfonyloxy, compound (VI) can be formed from compounds (VII) (wherein Y=OH and V=OH) and an appropriate derivatising agent, more particularly an appropriate sulphonylating agent such as arylsulfonylhalide, C₁-C₄ alkylsulfonylhalide, C₁-C₄ perfluoroalkylsulfonylhalide, quarternaryammonium C₁-C₄ alkylsulfonylhalide, halosulfonylhalide ,or an appropriate arylating agent such as arylhalide, or an appropriate acylating agent such as C₁-C₄ perfluoroalkanoylhalide, or C₁-C₄ alkanoylhalide), such as in an appropriate solvent. Preferably the halide substituent of the above is chloride, and preferably also the reaction takes place in the presence of a base. Compounds of formula (VII) (wherein Y=OH and V=OH) can be formed from compounds (VIII) and a hydrolising agent, preferably a hydroxide base (ideally 2 molar equivalents), more preferably a metal hydroxide such as sodium hydroxide, in an appropriate solvent, such as water. The metal of the hydroxide base can be as defined hereinbefore for Z (in ZOR). This will also apply for other reactions of Schemes 1, 2 and 3 hereafter where hydroxide base/hydrolising agent is used. Although the hydrolysing agent will normally act to introduce a hydroxide at D and at P, there may be some protecting groups within the scope of the invention which may not be hydrolysed, in which case a separate deprotecting agent can be used.

Compounds of formula (VI) where X = chloro, can be formed from (VII) wherein Y=Cl and V=P (such as OEt) (i.e. formula VIII) and a deprotecting agent.

Preferably the deprotecting agent as used herein in accordance with the invention is a hydrolysing agent, more preferably a hydroxide nucleophile, advantageously a hydroxide base (ideally 1 molar equivalent), such as sodium hydroxide preferably in an appropriate solvent, such as water.

Compounds of formula (VI) wherein X = diazonium, can formed from (VII) (wherein Y=NH₂, V=OH) and nitrous acid. Compounds of formula (VII) (wherein Y=NH₂, V=OH) can be formed from compounds of formula (VII) ( wherein Y=NH₂, V=P, e.g. OEt) and a deprotecting agent.

Intermediate (VII) (Y=NH₂, V=P, e.g. OEt) is formed from (VIII) and an ammoniating agent, such as ammonia, in an appropriate solvent, such as water.

Compounds of formula (VI) wherein X = (diarylsulfonyl)amino, can be formed from (VII) (wherein Y=NH₂, V=OH) and an appropriate derivatising agent , preferably an appropriate sulphonylating agent such as arylsulphonylhalide, preferably arylsulfonylchloride ( ideally at least 2 molar equivalents) preferably in the presence of a base (ideally 2 molar equivalents thereof), such as triethylamine in an appropriate solvent.

Compounds of formula (VI) wherein X is OR which is C₁₋₆ (preferably C₁-C₄) preferably primary or secondary alkoxy, can be formed from (VII) (wherein Y= C₁₋₆ (preferably C₁-C₄) primary or secondary alkoxy and V=P, (such as OEt) and a deprotecting agent.

Compounds of formula (VII) (wherein Y= C₁₋₆ (preferably C₁-C₄ ) primary or secondary alkoxy, V=P (e.g. OEt) can be formed by reacting a compound of formula (VIII) in the presence of ⁻OR, which is an appropriate alkoxide ( C₁₋₆ more preferably C₁-C₄ ) more preferably primary or secondary alkoxide), such as sodium ethoxide preferably in an appropriate solvent such as toluene.

Most preferably P=X (where X is an alkoxy) since this avoids transesterification issues.

The compounds of formula (VIII) can formed from compounds of formula (IX) by reaction with an amine NH(R3)(R4) optionally in the presence of a supplementary base (i.e. other than NH(R3)(R4)) preferably in an appropriate solvent , such as toluene. "D" in compounds (VIII) and (IX) is Cl or Br. Where a supplementary base is used it either does not react with the sulphonyl chloride moiety (such as a metal oxide, carbonate or bicarbonate) or it reacts with the sulphonyl chloride moiety in such a way as to keep it activated to nucleophilic attack (e.g. a tertiary amine such as triethylamine). The amine NH(R3)(R4) may also act as a base, in which case preferably more than one equivalent is present, more preferably about 2 equivalents (or more).

The compounds of formula (IX) can be formed from compounds of formula (X) in the presence of a chlorinating or brominating agent such as thionyl chloride or thionyl bromide more preferably in the presence of a halogenation catalyst, more preferably still thionyl chloride or thionyl bromide in the presence of dimethylformamide. The thionyl chloro/bromo can also act as the solvent, but more preferably the reaction takes place in an appropriate other solvent such as toluene. In such case only stoicheiometric amounts of thionyl chloride/bromide would be required, preferably at least 2 molar equivalents, more preferably at least 5 molar equivalents.

It is possible to undertake the four step conversion of (X) to (VI) (more particularly (XA) to (VIA) and (XB) to (VIB) ) in a single telescoped step, without intermediate product isolation, using the same solvent throughout (hereinafter the "telescoping solvent). Thus where X is an alkoxy, steps (X) to (VI) can be telescoped together using a single solvent such as a water immiscible inert organic solvent. More preferably a hydrocarbon solvent (such as toluene, xylenes, anisole, chlorobenzene, hexane, heptane, octane, nonane, decane, cyclohexane, methylcyclohexane) or ethers (such as dibutyl ether, diphenyl ether) or ketones (such as methylisobutylketone, methylethylketone) or esters (such as ethyl acetate, butyl acetate) or dimethylformamide. More preferably still a hydrocarbon solvent (such as toluene, xylenes, anisole, chlorobenzene, octane, nonane, decane, methylcyclohexane) or ethers (such as dibutyl ether, diphenyl ether) or esters (such as ethyl acetate, butyl acetate). More preferably still the telescoping solvent is toluene.

The intermediate of formula (X) is formed from a compound of formula (XI) or a salt thereof in the presence of a protecting forming agent which will form a protecting group (P) for the carboxylic acid (i.e. to form the -COP group). Preferably said protecting forming agent is an esterification agent, to form a carboxylic acid ester (wherein, e.g. P will be alkoxy and the protecting forming agent will be an alcohol) such as a C₁₋₆ carboxylic acid ester which will be carried through the reaction scheme and hydrolised under basic conditions to the carboxylic acid function of compound (VI). Most preferably the esterification agent is ethanol. An additional solvent such as toluene may be appropriate.

The intermediate of formula (XI) is formed from 2-hydroxynicotinic acid or a salt thereof in the presence of SO₃ ( ideally at least 1 molar equivalent of SO₃), for example using SO₃ in an aprotic solvent (e.g. nitrobenzene, nitromethane, 1,4-dioxane, dichloromethane) or in a mineral acid as solvent (e.g. sulphuric acid) or in a liquid carboxylic acid as solvent (e.g. acetic acid) or THF or heptane. More preferably still, the sulphonylating agent is oleum (SO₃in sulphuric acid) such as about 20% to 30% oleum.

Compound (III) is formed by reaction of intermediate (VI) and compound (V) in the presence of a coupling agent, such as N,N'-carbonyldiimidazole and a suitable solvent, such as ethyl acetate.

Compounds of the general formula (V) are prepared according to method shown in Example 1a to 1f hereinafter (i.e. for the formation of compound VA)

Scheme 2 illustrates a preferred embodiment for the formation of compound (VIA) where X is an alkoxy (and so Y in compound VIIA represents X), more preferably a C₁₋₆ primary or secondary alkoxy, such as ethoxy. However for other leaving groups the method for Scheme 1 would apply.

With reference to Scheme 2, the intermediate of formula (VIA) is formed from a compound of formula (VIIA) by removal of protecting group P by a deprotecting agent, advantageously by saponification in the presence of a hydrolising agent (as defined above for Scheme 1) such as sodium hydroxide, preferably in an appropriate solvent such as water and toluene.

The intermediate of formula (VIIA) is formed from a compound of formula (VIIIA) in the presence of an appropriate C₁₋₆ alkoxide (such as a primary or secondary alkoxide), preferably a metal alkoxide wherein the metal (Z) is as defined hereinbefore for ZOR such as sodium ethoxide, preferably in an appropriate solvent such as toluene or XH, wherein X is as defined hereinbefore. "D" in compounds (VIIIA) and (IXA) is Cl or Br.

The intermediate of formula (VIIIA) is formed from a compound of formula (IXA) by reaction with N-ethylpiperazine in the presence of a base, such as triethylamine, preferably in an appropriate solvent such as toluene.

The intermediate of formula (IXA) is formed from a compound of formula (XA) in the presence of a chlorinating or brominating agent as defined for the same step in Scheme 1, most preferably thionyl chloride or bromide / dimethylformamide.

The intermediate of formula (XA) is formed from a compound of formula (XI) in the presence of an agent which will form a protecting group (P) for the carboxylic acid (i.e. to form the -COP group) as defined hereinbefore. Most preferably the esterification agent is ethanol. An additional solvent such as toluene may be appropriate.

The intermediate of formula (XI) is formed from 2-hydroxynicotinic acid with a sulphonylating agent (as defined hereinbefore) such as 20 to 30% oleum.

Again the four step conversion of (XA) to (VIA) can be telescoped together (as set out hereinbefore), without intermediate product isolation, using the same solvent. The list of solvents described with respect to Scheme 1 are directly applicable here. Most preferably the solvent is toluene.

For example after formation of compound (IXA), the excess chlorinating/brominating agent could be azeotroped off at the azeotrope temperature of the said agent and the telescope solvent. After formation of compound (VIIIA), the HBr/HCl (i.e. HD) salts which are formed could be washed out (in aqueous) or filtered from the reaction vessel and the remainder of the aqueous solvent azeotroped off with some of the telescoping solvent. In the formation of compound (VIIA), if the alkoxide used to introduce X is dissolved in solvent (such as ethanol), then this solvent could again be azeotroped off with some of the telescoping solvent prior to formation of compound (VIA) to facilitate isolation. If solid alkoxide is used then this latter azeotroping step is not required.

Most preferably the telescoping solvent for any telescoped steps of Scheme 1 and more particularly Schemes 2 and 3, is toluene.

Compound (IIIA) is formed by reaction of intermediate (VIA) and 4-Amino-5-ethyl-1 (2-pyridylmethyl)-1H-pyrazole-3-carboxamide (compound VA) in the presence of a coupling agent, such as 1-(3-dimethylaminopropyl)-3-ethyl carbodiimide hydrochloride and where desirable also in the presence of a base and/or an accelerator. In one example of a coupling system, the carboxylic acid function of (VIA) is first of all activated using molar equivalent of a reagent such as N,N^{'}-carbonyldimidazole (as coupling agent) in a suitable solvent, e.g. ethyl acetate, at from about room temperature to about 80ºC, followed by reaction of the intermediate imidazolide with (IXA) at from about 35 to about 80ºC. In another example, intermediate (VIA) could be coupled to the pyrazole (VA) in the presence of 1-hydroxybenzotriazole, triethylamine and 1-(3-dimethylaminopropyl)-3-ethyl-carbodiimide hydrochloride.

Compound (IB) (Scheme 3) wherein X also represents a C₁₋₆ alkoxy (preferably a primary or secondary alkoxy, such as ethoxy) can be formed in an analogous fashion to that of compound (IA). The reagents etc. for Scheme 2 are also directly applicable to Scheme 3.

It will be appreciated that salts of the compounds of Schemes 1 to 3 can be formed in accordance with the invention by converting the relevant compound to a salt thereof (either *in situ* or as a separate step). For example base addition salts of the compounds of formulae (VI) and (XI) can be formed and are in accordance with the invention. Also an acid addition salt of the compound of formula (I) can be formed in accordance with the invention.

By way of illustration, acid addition salts of compounds of formula (I) (more particularly IA and IB) can be formed by reacting a compound of formula (I) with an equimolar or excess amount of acid. The salt may then be precipitated out of solution and isolated by filtration or the solvent can be stripped off by conventional means. Typical salts which can be used in the Schemes of 1 to 3 are given in PCT/IB99/00519. An example of a salt of compounds IA and IB is the tosylate and besylate respectively.

Suitable protecting groups for use in accordance with the invention can be found in "Protecting Groups" edited by P.J. Kocienski, Thieme, New York, 1994 - see particularly chapter 4, page 118-154 for carboxy protecting groups; and "Protective Groups in Organic Synthesis" 2^{nd} edition, T.W. Greeene & P.G.M. Wutz, Wiley - Interscience (1991)- see particularly chapter 5 for carboxy protecting groups.

The invention will now be described by way of example only with reference to the following examples.

### Example 1

### (1a) Ethyl 3-ethyl-1H-pyrazole-5-carboxylate

Ethanolic sodium ethoxide solution (21% w/w; 143 ml, 0.39 mol) was added dropwise to a stirred, ice-cooled solution of diethyl oxalate (59.8 ml, 0.44 mol) in absolute ethanol (200 ml) under nitrogen and the resulting solution stirred for 15 minutes. Butan-2-one (39 ml, 0.44 mol) was then added dropwise, the cooling bath removed, the reaction mixture stirred for 18 hours at room temperature and then for 6 hours at 40°C, then the cooling bath reintroduced. Next, glacial acetic acid (25 ml, 0.44 mol) was added dropwise, the resulting solution stirred for 30 minutes at 0°C, hydrazine hydrate (20 ml, 0.44 mol) added dropwise, then the reaction mixture allowed to warm to room temperature and maintained there over a period of 18 hours, before being evaporated under reduced pressure. The residue was partitioned between dichloromethane (300 ml) and water (100 ml), then the organic phase separated, washed with water (2 x 100ml), dried (Na₂SO₄) and concentrated under reduced pressure to give the title compound (66.0 g). δ (CDCl₃): 1.04 (3H,t), 1.16 (3H,t), 2.70 (2H,q), 4.36 (2H,q), 6.60 (1H,s). LRMS: m/z 169 (M+1)⁺.

### (1b) 3-Ethyl-1H-pyrazole-5-carboxylic acid

Aqueous sodium hydroxide solution (10M; 100 ml, 1.0 mol) was added dropwise to a stirred suspension of the title compound of example (4a) (66.0 g, 0.39 mol) in methanol and the resulting solution heated under reflux for 4 hours. The cool reaction mixture was concentrated under reduced pressure to ca. 200 ml, diluted with water (200 ml) and this mixture washed with toluene (3 x 100 ml). The resulting aqueous phase was acidified with concentrated hydrochloric acid to pH 4 and the white precipitate collected and dried by suction to provide the title compound (34.1 g). δ (DMSO_{d6}): 1.13 (3H,t), 2.56 (2H,q), 6.42 (1H,s).

### (1c) 3-Ethyl-4-nitro-1H-pyrazole-5-carboxylic acid

Fuming sulphuric acid (17.8 ml) was added dropwise to stirred, ice-cooled fuming nitric acid (16.0 ml), the resulting solution heated to 50°C, 3-ethyl-1H-pyrazole-5-carboxylic acid added portionwise over 30 minutes whilst maintaining the reaction temperature below 60°C. The resulting solution was heated for 18 hours at 60°C, allowed to cool, then poured onto ice. The title compound was obtained as a brown solid (64%). δ (DMSO_{d6}): 1.18 (3H,t), 2.84 (2H,m), 13.72 (1H,s).

### (1d) 3-Ethyl-4-nitro-1H-pyrazole-5-carboxamide

A solution of the title compound of example (1c) (15.4 g, 0.077 mol) in thionyl chloride (75 ml) was heated under reflux for 3 hours and then the cool reaction mixture evaporated under reduced pressure. The residue was azeotroped with tetrahydrofuran (2 x 50 ml) and subsequently suspended in tetrahydrofuran (50 ml), then the stirred suspension ice-cooled and treated with gaseous ammonia for 1 hour. Water (50 ml) was added and the resulting mixture evaporated under reduced pressure to give a solid which, after trituration with water and drying by suction, furnished the title compound as a white solid (90%). δ (DMSO_{d6}): 1.17 (3H,t), 2.87 (2H,m), 7.40 (1H,s), 7.60(1H,s), 7.90 (1H,s). LRMS: m/z 185 (M+1)⁺.

### (1e) 5-Ethyl-4-nitro-1-(2-pyridylmethyl)-1H-pyrazole-3-carboxamide.

Caesium carbonate (1.414 kg, 4.34mol) was added to a suspension of the title compound of example (1d) (800g, 4.34mol) in acetonitrile (51) and the mixture warmed to 60°C. 2-Chloromethylpyridine (664.7g, 5.23mol) was added and the reaction heated at 70°C for 7 hours, then water (9.5l) added and the reaction mixture cooled to 10°C. Granulation of this mixture gave a precipitate which was filtered and dried to afford 3-ethyl-4-nitro-1-(pyridin-2-yl)methyl-pyrazole-5-carboxamide (367g). Sodium chloride (1.58 kg) was added to the filtrate and the solution extracted with ethyl acetate (4 x 1.75l). The combined organic extracts were distilled to remove approximately 10 l of solvent, toluene (5.6l) added over 35 minutes to the hot (69-76°C) solution and the mixture allowed to cool. The resulting suspension was granulated at <10°C for 30 minutes, filtered, the solid washed with ethyl acetate:toluene (50:50) 600 ml) and dried (60°C) to afford the title compound (624g 52%) as a light brown solid. δ (DMSO_{d6}): 1.08 (3H,t), 3.02 (2H,q), 5.53 (2H,s), 7.34 (2H,m), 7.65 (1H,s), 7.82 (1H,m), 7.93 (1H,s), 8.52 (1H,d). LRMS: m/z 276 (M+1)⁺.

### (1f) 4-Amino-5-ethyl-1-(2-pyridylmethyl)-1H-pyrazole-3-carboxamide. (Compound VA)

A mixture of Lindlar catalyst (2g) and the title compound of example (1e) (20g, 72.7mmol) in ethanol (160ml) was hydrogenated for 48 hours at 345kPa (50psi) and 50°C, then cooled and filtered. The filtrate was combined with an IMS wash (50ml) of the filter pad and concentrated under reduced pressure to a colume of 100ml. The remaining ethanol was removed by distillation, and replaced with ethyl acetate until a head temperature of 77°C had been achieved. The cooled mixture was granulated at 4°C, filtered and dried to afford the title compound (13.17g, 73%) as a light brown solid. δ (DMSO_{d6}): 0.90 (3H,t), 2.54 (2H,q), 4.48 (2H,s), 5.31 (2H,s), 6.89 (1H,d), 6.95 (1H,s), 7.11 (1H,s), 7.28 (1H,m), 7.74 (1H,m), 8.50 (1H,d). LRMS: m/z 246 (M+1)⁺.

### (1g) 2-Hydroxy-5-sulfonicotinic acid (Compound XIA)

2-Hydroxynicotinic acid (27Kg, 194.2mol) was added portionwise to 30% oleum (58.1 Kg) at 50°C over 1hr. This caused an exotherm to 82°C. The reaction mixture was heated further to 140°C. After maintaining this temperature for 12hrs the reactor contents were cooled to 15C and filtered. The filter cake was then re-slurried with acetone (33Kg) at room temperature, filtered and dried to afford the title compound (35.3Kg, 83%) as a white solid. Decomposition pt 273°C. δ (DMSO_{d6}): 7.93 (1H, d), 8.42 (1H, d). m/z (Found:220 [M+H]⁺, 100%. C₆H₆NO₆S requires 220).

### (1h) Ethyl 2-hydroxy-5-sulfonicotinoate (Compound XA)

2-Hydroxy-5-sulfonicotinic acid (XIA) (500g, 2.28mol) was dissolved in ethanol (2.5L) with stirring and heated to 80°C. After 30mins 0.5L of solvent was distilled off, then replaced with fresh ethanol (0.5L) and taken back to 80°C. After a further 60mins 1.0L of solvent was distilled off, then replaced with fresh ethanol (1.0L) and taken back to 80°C. After a further 60mins 1.0L of solvent was distilled off, the reaction cooled to 22°C and stirred for 16hr. The precipitated product was filtered, washed with ethanol (0.5L) and dried at 50°C under vacuum to afford the title compound (416g, 74%) as a white solid. Decomposition pt 237°C. δ (DMSO_{d6}): 1.25 (3H, t), 4.19 (2H,q), 7.66 (1H, d), (1H, d). m/z (Found:248 [M+H]⁺, 100%. C₈H₁₀NO₆S requires 248).

### (1i) Ethyl 2-chloro-5-chlorosulfonylnicotinoate(Compound IXA)

Ethyl 2-hydroxy-5-sulfonicotinoate (XA) (24.7g, 0.1 mol) was slurried in thionyl chloride (238g, 2.0mol) and dimethylformamide (1.0mL) with stirring. The reaction mixture was then heated to reflux for 2.5hr. The bulk of the thionyl chloride was removed under vacuum with residual thionyl chloride removed with a toluene azeotrope to afford the crude title compound (30.7g, 108%) as a yellow oil. δ (CDCl₃): 1.46 (3H, t), 4.50 (2H, q), 8.72 (1H, d), 9.09 (1H, d). This was taken directly onto the next step.

### (1j) Ethyl 2-chloro-5-(4-ethyl-1-piperazinylsulfonyl)nicotinoate (Compound VIIIA)

Crude ethyl 2-chloro-5-chlorosulfonicotinoate (IXA) (30.7g, 0.1 mol assumed) was dissolved in ethyl acetate (150mL) with stirring then ice cooled. To this was added a solution of N-ethylpiperazine (11.4g, 0.1mol) and triethylamine (22.5g, 0.22mol) in ethyl acetate (50mL), carefully over 30mins, keeping the internal temperature below 10°C. Once the addition was complete the reaction was allowed to warm to 22°C and stir for 1 hr. The solid was filtered off and the remaining filtrate was concentrated under vacuum to afford the crude title compound (37.1g, 103%) as a crude yellow gum. δ (CDCl₃): 1.10 (3H, t), 1.42 (3H, m), 2.50 (2H, m), 2.60 (4H, m), 3.19 (4H, m), 4.43 (2H, q), 8.40 (1H, d), 8.80 (1H, d). m/z (Found:362 [M+H]⁺, 100%. C₁₄H₂₁CIN₃O₄S requires 362).

### (1k) Ethyl 2-ethoxy-5-(4-ethyl-1-piperazinylsulfonyl)nicotinoate (Compound VIIA X=OEt)

A solution of ethyl 2-chloro-5-(4-ethyl-1-piperazinylsulfonyl)nicotinoate (VIIIA) (36.1g, 0.1mol) in ethanol (180mL) was cooled to 10°C with stirring. Sodium ethoxide (10.2g, 0.15mol) was added portionwise keeping the temperature below 20°C. The reaction mixture was then stirred at ambient temperature for 18 hours. The precipitate was filtered off and water (180mL) added to the filtrate. The filtrate was then heated to 40°C for 1 hour. Ethanol (180mL) was then distilled off at ambient pressure and the remaining aqueous solution allowed to cool to ambient temperature. The precipitated product was then filtered off, washed with water and dried under vacuo at 50°C to afford the title compound (12.6g, 34%) as a light brown solid. M.pt. 66-68°C. δ (CDCl₃): 1.04 (3H, t), 1.39 (3H, t), 1.45 (3H, t), 2.41 (2H, q), 2.52 (4H, m), 3.08 (4H, m), 4.38 (2H, q), 2.57 (2H, q), 8.38 (1H, d), 8.61 (1H, d). m/z (Found:372 [M+H]⁺, 100%. C₁₆H₂₆N₃O₅S requires 372).

### (1l) 2-Ethoxy-5-(4-ethyl-1-piperazinylsulfonyl)nicotinic acid (Compound VIA; X=OEt)

Ethyl 2-ethoxy-5-(4-ethyl-1-piperazinylsulfonyl)nicotinoate (VIIA) (10.2g, 0.0275mol) was dissolved in toluene (50mL) and a solution of sodium hydroxide (1.1g, 0.0275mol) in water (20mL) added to it. This two phase mixture was then stirred vigorously at ambient temperature overnight. The aqueous phase was separated off and adjusted to pH=5.6 by addition of conc. hydrochloric acid. The precipitated product was slurried with ice cooling for 15minutes, filtered, water washed and dried under vacuo at 50°C to afford the title compound as an off-white solid. Mpt 206-207°C. δ (CDCl₃): 1.25 (3H, t), 1.39 (3H, t), (2H, q), 3.03 (4H, m), 3.25 (4H, m), 4.50 (2H, q), 8.25 (1H, d), 8.56 (1H, d). m/z (Found:344 [M+H]⁺, 100%. C₁₄H₂₂N₃O₅S requires 344).

This step (1l) is already set out in preparation 23 of PCT/IB99/00519 (herein incorporated by reference) and the yield obtained is 88%.

### (1m) N-[3-carbamoyl-5-ethyl-1-(2-pyridylmethyl)-1H-pyrazol-4-yl]-2-ethoxy-5-(4-ethyl-1-piperazinylsulfonyl)nicotinamide (Compound IIIA; X=OEt)

2-Ethoxy-5-(4-ethyl-1-piperazinylsulfonyl)nicotinic acid (VIA) (0.875Kg, 2.55mol) was charged followed by ethyl acetate (7L, 8ml/g) to the reaction vessel and 2ml/g was distilled off at atmospheric pressure to ensure the reaction system was dry. The slurry was cooled to room temperature under a nitrogen atmosphere and carbonyldiimidazole (0.43Kg, 2.65mol) added in one portion. The slurry was heated to 35°C and held for half an hour. The reaction was further heated to 45-50°C and held for a further half hour. The reaction was then heated to reflux, stirring at reflux for one hour. On confirmation of complete imidazolide formation the reaction was cooled to 45-50°C under nitrogen and 4-amino-5-ethyl-1-(2-pyridylmethyl)-1H-pyrazole-3-carboxamide (VA) (0.59Kg, 2.42mol) charged in one portion before returning to reflux and a further 1ml/g was distilled off at atmospheric pressure. The reaction was stirred at reflux for 16 hours . The reaction was cooled to 10-15°C and granulated for one hour. The reaction slurry was filtered and washed (ethyl acetate) which was dried under vacuum at 50°C to afford the title compound (1.252Kg, 90.7%) as a white solid. Mpt 178-179°C. δ (CDCl₃): 1.04 (3H, t), 1.06 (3H, t), 1.59 (3H, t), 2.40 (2H, q), 2.50 (4H, m), 2.90 (2H, q), 3.08 (4H, m ), 4.78 (2H, q), 5.35 (1H, s), 5.48 (2H, s), 6.68 (1H, s), 6.92 (1H, d), 7.22 (1H, m), 7.65 (1H, m), 8.58 (1H, d), 8.64 (1H, d), 8.83 (1H, d). m/z (Found:571 [M+H]⁺, 100%. C₂₆H₃₅N₈O₅S requires 571).

### (1n) 4-{6-Ethoxy-5-[3-ethyl-6,7-dihydro-7-oxo-2-(2-pyridylmethyl)-2H-pyrazolo[4,3-d]pyrimidin-5-yl]-3-pyridylsulfonyl}-1-ethylpiperazine (Compound IIA; X=OEt)

Potassium ethoxide solution (86g, 0.25mol, 24%w/w in ethanol) was charged to the vessel and ethanol (235mL) added. Ethyl acetate (10.8g) was added to reaction mixture at ambient. N-[3-carbamoyl-5-ethyl-1-(2-pyridylmethyl)-1H-pyrazol-4-yl]-2-ethoxy-5-(4-ethyl-1-piperazinylsulfonyl)nicotinamide (IIIA) (70g, 0.122mol) was added in one portion to the solvent mixture and the reaction was stirred at ambient. The reaction mixture was warmed in a sealed vessel to a temperature of 120°C, producing an internal pressure of approximately 50-60 p.s.i., the pressure was then made up to 80 p.s.i by applying a nitrogen pressure and the reaction was stirred for 8 hours. The reaction was cooled after 8 hours and ethanol is then reduced under atmospheric distillation to a volume of about 720ml (3ml/g). Ethyl Acetate (840ml) was added to the ethanol solution and then reduced under atmospheric distillation to a volume of 1920ml (8ml/g). Dilute aqueous hydrochloric acid was added to adjust the pH of the reaction mixture from about pH=13 to pH=8. This was added over 30 min. The mixture was stirred for 5 minutes, warmed to 50°C and the phases were separated. Water (140mL) was added to ethyl acetate layer, stirred, warmed to 50°C and the phases were separated. The ethyl acetate phase was cooled from 50°C to 0-5°C over two hours, and stirred for a further hour, the solid filtered off and washed with ethyl acetate (3) 0-5°C and dried under vacuum at 60°C to afford the title compound (83%) as a white solid. Mpt 178-180°C. δ (CDCl₃): 1.02 (3H, t), 1.30, 3H, t), 1.58 (3H, t), 2.41 (2H, q), 2.55 (4H, m), 3.04 (2H, q), 3.10 (4H, m), 4.75 (2H, q), 5.69 (2H, s), 7.10 (1H, d), 7.22 (1H, m), 7.63 (1H, m), 8.57 (1H, d), 8.63 (1H, d), 9.02 (1H, d). m/z (Found:553 [M+H]⁺, 100%. C₂₆H₃₂N₈O₄S requires 553).

### (1p) 1-Ethyl-4-{5-[3-ethyl-6,7-dihydro-7-oxo-2-(2-pyridylmethyl)-2H-pyrazolo[4,3-d]pyrimidin-5-yl]-6-(2-methoxyethoxy)-3-pyridylsulfonyl}piperazine (Compound IA from Compound IIA, X = OEt)

4-{6-Ethoxy-5-[3-ethyl-6,7-dihydro-7-oxo-2-(2-pyridylmethyl)-2H-pyrazolo[4,3-d]pyrimidin-5-yl]-3-pyridylsulfonyl}-1-ethylpiperazine (IIA) (100g, 0.18mol) was added in one portion to 2-methoxyethanol (600mL) and the reaction was stirred at ambient for 30 minutes to produce a heterogeneous mixture. To the mixture was added 2-methoxyethyl acetate (42.8g, 0.36mol). Potassium *t*-butoxide (30.52g, 0.27mol) was added in one portion to the stirred reaction mixture, this was found to produce an exotherm of 20-30°C. The reaction was stirred until a steady temperature was obtained. The reaction mixture was heated to reflux temperature (115-125)°C and maintained at reflux temperature for 15 minutes. The temperature at reflux should be 123-125°C, if not, distill off solvent until the temperature is in the required range and maintain this reflux temperature for 4 hours. The reaction was then cooled to 60°C and the remaining solvent was reduced under vacuum distillation to a volume of 3ml/g. The viscous solution was stirred and cooled to ambient temperature. Water (533mL) was added to the viscous solution and stirred to produce a homogenous solution. Water (266mL) and conc hydrochloric acid (25.82g) were premixed and added over 1 hour to adjust the pH of the reaction mixture from about pH=13 to pH=8. The mixture was cooled from ambient to 0-5°C and stirred for a further hour, filtered off the solid, washed with water (200mL) and dried under vacuum at 60°C to afford the title compound (98.12g, 92.7%) as a white solid. Mpt 157-158°C. δ (CDCl₃): 1.02 (3H, t), 1.30 (3H, t), 2.40 (2H, q), 2.55 (4H, m), 3.03 (2H, q), 3.12 (4H, m), 3.55 (3H, s), 3.85 (2H, m), 4.77 (2H, m), 5.66 (2H, s), 7.10 (1H, d), (1H, m), 7.63 (1H, m), 8.57 (1H, d), 8.62 (1H, d), 8.97 (1H, d). (Found:583 [M+H]⁺, 100%. C₂₇H₃₅N₈O₅S requires 583).

### Example 2

### 1-Ethyl-4-{5-[3-ethyl-6,7-dihydro-7-oxo-2-(2-pyridylmethyl)-2H-pyrazolo[4,3-d]pyrimidin-5-yl]-6-(2-methoxyethoxy)-3-pyridylsulfonyl}piperazine (IA) from N-[3-carbamoyl-5-ethyl-1-(2-pyridylmethyl)-1H-pyrazol-4-yl]-2-ethoxy-5-(4-ethyl-1-piperazinylsulfonyl)nicotinamide (Compound IIIA, X = OEt)

N-[3-carbamoyl-5-ethyl-1-(2-pyridylmethyl)-1H-pyrazol-4-yl]-2-ethoxy-5-(4-ethyl-1-piperazinylsulfonyl)nicotinamide (IIIA) (11.41g, 0.02mol) was added under nitrogen in one portion to 2-methoxyethanol (45mL) and the reaction was stirred at ambient temperature for 10 minutes. Ethyl pivalate (6.5g, 0.05mol) was then washed in with 2-methoxyethanol (5mL). Potassium *tert*-butoxide (5.4g, 0.048mol) was added in portionwise over 10 minutes to the stirred reaction mixture, this was found to produce an exotherm (of 20-50°C). The reaction was stirred until a steady temperature was obtained. The reaction mixture was a clear pale yellow solution. The reaction mixture was heated to reflux temperature (115-125°C) and maintained at reflux temperature for five hours. The remaining solvent is then reduced under vacuum distillation to a volume of 2ml/g. The viscous solution was stirred and cooled to ambient temperature. Water (70mL, 6mL/g) was added to the viscous solution and stirred to produce a homogenous solution. Dilute aqueous hydrochloric acid was added to adjust the pH of the reaction mixture from about pH=13 to pH=8. This was a slow addition over one hour. The precipitated product suspension was ice-cooled and granulated for 1 hour, filtered, washed with water and dried under vacuum at 50°C to afford the title compound (9.7g, 83.3%) as a white solid. Mpt 157-158°C. δ (CDCl₃): 1.02 (3H, t), 1.30 (3H, t), 2.40 (2H, q), 2.55 (4H, m), 3.03 (2H, q), 3.12 (4H, m), 3.55 (3H, s), 3.85 (2H, m), 4.77 (2H, m), 5.66 (2H, s), 7.10 (1H, d), 7.21 (1H, m), 7.63 (1H, m), 8.57 (1H, d), 8.62 (1H, d), 8.97 (1H, d). m/z (Found:583 [M+H]⁺, 100%. C₂₇H₃₅N₈O₅S requires 583).

The above reaction could also be repeated with different hydroxide trapping agents. If the ethyl pivalate (2.5 molar equivalents) was switched for 2-methoxyethylacetate (2.5 molar equivalents) or 2-methoxypivalate (2.5molar equivalents) the resulting yield was 76.3% and 84.8% respectively.

### Example 3

### 1-Ethyl-4-{5-[3-ethyl-6,7-dihydro-7-oxo-2-(2-pyridylmethyl)-2H-pyrazolo[4,3-d]pyrimidin-5-yl]-6-(2-methoxyethoxy)-3-pyridylsulfonyl}piperazine (Compound IA) from N-[3-carbamoyl-5-ethyl-1-(2-pyridylmethyl)-1H-pyrazol-4-yl]-2-ethoxy-5-(4-ethyl-1-piperazinylsulfonyl)nicotinamide (Compound IIIA, X = OEt)

N-[3-carbamoyl-5-ethyl-1-(2-pyridylmethyl)-1H-pyrazol-4-yl]-2-ethoxy-5-(4-ethyl-1-piperazinylsulfonyl)nicotinamide (IIIA) (500g, 0.876mol) was added under nitrogen in one portion to 2-methoxyethanol (2.5L, 5mL/g) and the reaction was stirred at ambient temperature for 10 minutes. Potassium *tert*-butoxide (236g, 2.103 mol) was added in portionwise over 10 minutes to the stirred reaction mixture, this was found to produce an exotherm (of 20-50°C). The reaction was stirred until a steady temperature was obtained. The reaction mixture was a clear pale yellow solution. Ethyl pivalate (285g, 2.190mol) was then washed in with 2-methoxyethanol (0.3L). The reaction mixture was heated to reflux temperature (115-125°C) and maintained at reflux temperature for six hours. The remaining solvent is then reduced under vacuum distillation to a volume of 2ml/g. The viscous solution was stirred and cooled to ambient temperature. Water (3L, 6mL/g) was added to the viscous solution and stirred to produce a homogenous solution. Dilute aqueous hydrochloric acid was added to adjust the pH of the reaction mixture from about pH=13 to pH=8. This was a slow addition over one hour. Methylisobutylketone (3L) was then added and the mixture heated to 55°C. The lower aqueous layer was separated off whilst the remaining organic laver was washed with warm water (500mL). The organic layer was taken and 1 L distilled out under vacuum. The remaining solution was cooled to 50°, the resulting precipitate being granulated for 1 hour, filtered, washed with methylisobutylketone (1L) and dried under vacuum at 50°C to afford the title compound (400.3g, 78.4%) as a white solid. Mpt 157-158°C. δ (CDCl₃): 1.02 (3H, t), 1.30 (3H, t), 2.40 (2H, q), 2.55 (4H, m), 3.03 (2H, q), 3.12 (4H, m), 3.55 (3H, s), 3.85 (2H, m), 4.77 (2H, m), 5.66 (2H, s), 7.10 (1H, d), 7.21 (1H, m), 7.63 (1H, m), 8.57 (1H, d), 8.62 (1H, d), 8.97 (1H, d). m/z (Found:583 [M+H]⁺, 100%. C₂₇H₃₅N₈O₅S requires 583).

### Example 4

### 2-Ethoxy-5-(4-ethyl-1-piperazinylsulfonyl)nicotinic acid (Compound VIA) - Telescoped process in toluene from ethyl 2-hydroxy-5-sulfonicotinoate (Compound XA, X = OEt)

In a particularly preferred embodiment of the invention compound XA is formed by a telescoped process thereby further reducing the overall number of steps to form compound IA from commercially available starting materials.

Ethyl 2-hydroxy-5-sulfonicotinoate (XA) (441.5g, 1.79mol) was dissolved in toluene (1.77L) and thionyl chloride (1.06Kg, 8.93mol) and dimethylformamide (71.3mL) were then added. The stirred suspension was then heated to reflux for 3 hours to yield a yellow solution. Thionyl chloride (2.87L) was then distilled with continual replacement with toluene (2.15L). The pale yellow solution was then cooled to 10°C and a stirred solution of N-ethylpiperazine (198.9g, 1.66mol) and triethylamine (392.2g, 3.88mol) in toluene (700mL) added drop-wise over 90 minutes keeping the reaction mixture below 10°C . The reaction was stirred at ambient temperature for 18 hours then washed with water (2 x 700mL) and brine (2 x 350mL). The toluene phase was azeotropically dried by distilling off 1750mL which was continuously replaced by dry toluene (1750mL). The remaining brown solution was cooled to 10°C and sodium ethoxide (178.0g, ) was added portion-wise keeping the temperature below 10°C. The reaction was then stirred at 10°C for 1 hour then allowed to warm to ambient temperature and stirred for 18 hours. Sodium hydroxide (34.9g) dissolved in water (1.5L) was then added to the toluene mixture and the 2 phase mixture was vigorously stirred for 18 hours at 40°C. Once cooled to ambient temperature the aqueous phase was separated off. To this was added conc. hvdrochloric acid to pH=3 which precipitated a light brown solid which was granulated for 2 hour with ice cooling. The precipitate was filtered washed with water (300mL) and dried under vacuo at 50°C to afford the title compound (338.4g, 57.4%) as an off-white solid. Mpt 206-207°C. δ (CDCl₃): 1.25 (3H, t), 1.39 (3H, t), 2.82 (2H, q), 3.03 (4H, m), 3.25 (4H, m), 4.50 (2H, q), 8.25 (1H, d), 8.56 (1H, d). m/z (Found:344 [M+H]⁺, 100%. C₁₄H₂₂N₃O₅S requires 344).

### Example 5

### (5a) N-[3-carbamoyl-5-ethyl-1-(2-pyridylmethyl)-1H-pyrazol-4-yl]-5-(4-ethyl-1-piperazinylsulfonyl)-2-(2-methoxyethoxy) nicotinamide (Compound IVA)

5-(4-ethyl-1-piperazinylsulfonyl)-2-(2-methoxy)nicotinic acid (37.3g, 0.1 mol) was prepared from the free base of preparation 29 of PCT/IB/00519 and was charged followed by ethyl acetate (7L, 8ml/g) to the reaction vessel and 2ml/g of ethyl acetate was distilled off at atmospheric pressure to ensure the reaction system was dry. The slurry was cooled to room temperature under a nitrogen atmosphere and carbonyldiimidazole (16.87g, 0.104mol) added in one portion. The slurry was heated to 35°C and held for 30 mins. The reaction was further heated to 45-50°C and held for a further 30mins. The reaction was then heated to reflux, stirring at reflux for one hour. On confirmation of complete imidazolide formation the reaction was cooled to 40-43°C under nitrogen and 4-amino-5-ethyl-1-(2-pyridylmethyl)-1H-pyrazole-3-carboxamide (preparation 40 of PCT/IB99/00519; 0.59Kg, 2.42mol) was then charged in one portion before returning to reflux and a further 1 ml/g was distilled off at atmospheric pressure. The reaction was stirred at reflux for 20 hours. The reaction was evaporated to yield an oil which crystallised on standing overnight. The resulting solid was dissolved in 5ml/g dichloromethane and washed with 5ml/g water. The organic layer was evaporated to yield the titled compound (42g, 70% yield), as a white crystalline solid. Mpt 145-148°C. δ (CDCl₃): 1.02 (3H, t), 1.07 (3H, t), 1.64 (3H, s), 2.39 (2H, q), 2.51 (4H, t), 2.85 (2H, q), 3.09 (3H, t) 3.40 (3H, s), 3.95 (2H, t) 4.85 (2H, t), 5.34 (1H, s), 5.47 (2H, s), 6.68 (1H, s), 6.90 (1H, d), 7.23 (1H, m), 7.65 (1H, m), 8.58 (1H, d), 8.64 (1H, d), 8.83 (1H, d), 10.48 (1H, s). m/z (Found:600.9 [M+H]⁺, 100%. C₂₇H₃₆N₈O₆S requires 600.7).

### (5b) 1-ethyl-4-{5-[3-ethyl-6,7-dihydro-7-oxo-2-(2-pyridylmethyl)-2H-pyrazolo[4,3-d]pyrimidin-5-yl]-1,2-dihydro-6-(2-methoxyethoxy)-3-pyridylsulfonyl}piperazine (Compound IA)

N-[3-carbamoyl-5-ethyl-1-(2-pyridylmethyl)-1H-pyrazol-4-yl]-5-(4-ethyl-1-piperazinylsulfonyl)-2-(2-methoxyethoxy) nicotinamide (4.1 g, 6.8 mmol) was charged followed by 3-methyl-3-pentanol (21mls, 5ml/g), potassium t-butoxide (1.5g, 13.6 mmol) and methoxyethyl pivalate (2.18g, 13.6 mmol) in that order to form a solution and then heated to reflux. After 20 hours at reflux a liquid chromatography sample indicated 70% of the title compound had been formed (consistent with reference standard of the title compound which was verified by LCMS) (Found: 582.96 ,100% C₂₇H₃₄N₈O₅S requires 582.69).

### Example 6

### (6a) 5-Ethyl-1-methyl-4-nitro-1H-pyrazole-3-carboxamide.

A slurry of 3-ethyl-4-nitro-1*H*-pyrazole-5-carboxamide (100 g, 0.54 Mol) in acetone (1 L) at room temperature was treated with potassium carbonate (150 g, 1.08 Mol) in water (0.7 L) to form a yellow solution. The solution was then treated with methyl iodide (37 ml, 0.58 Mol) and the reaction allowed to stir for approximately 48 hours. The reaction was filtered and the liquors separated discarding the aqueous layer and concentrating the organic phase to precipitate a solid. The slurry was cooled, granulated, filtered and washed with acetone (100 mL). The resultant solid was dried under vacuum to yield a white solid, 47.5 g, 39%. m.p. = 188°C. δ (DMSO-d₆): 1.19 (3H, t), 2.95 (2H, q), 3.85 (3H, s), 8.31 (2H, s, br). m/z = 199 [M+H]⁺, C₇H₁₀N₄O₃ requires 198.18.

### (6b) 4-Amino-5-ethyl-1-methyl-1H-pyrazole-3-carboxamide (Compound VB).

5-Ethyl-1-methyl-4-nitro-1*H*-pyrazole-3-carboxamide (1.0 Kg, 5.05 Mol) was added to (15 L) IMS and the mixture hydrogenated at room temperature, 55 p.s.i. overnight over 5% Pd/C (15% w/w). The catalyst was filtered off and the filtrate evaporated to yield a solid which was slurried in *iso*-propyl alcohol (750 mL), heated to reflux, cooled to ambient, filtered and washed with *iso*-propyl alcohol (1 L). The solid was dried in vacuo overnight to yield 529 g, 84.5% of an off white to pink solid. m.p. = 155°C. δ (CDCl₃): 1.19 (3H, t), 2.60 (2H, q), 3.76 (3H, d), 3.96 (2H, s), 5.27 (1H, s) 6.55 (1H, s). m/z = 169 [M+H]⁺, 100%, C₇H₁₂N₄O requires 168.2.

### (6c) N-(3-Carbamoyl-5-ethyl-1-methyl-1H-pyrazol-4-yl)-2-ethoxy-5-(4-ethyl-1-piperazinylsulfonyl)nicotinamide (Compound IIIB).

2-Ethoxy-5-(4-ethyl-1-piperazinylsulfonyl)nicotinic acid (17.3 g, 0.05 Mol) was charged to the reaction vessel followed by ethyl acetate (137 mL) and 2ml/g was distilled off at atmospheric pressure to ensure the system was dry. The slurry was cooled to room temperature under a nitrogen atmosphere and 1,1-carbonyldiimidazole (8.51 g, 52.0 mMol) added in one portion. The slurry was heated to 35°C and held for half an hour. The reaction was heated to 45-50°C and held for a further 30 min. The reaction was then heated to reflux, stirring at reflux for 0.5 hours. On confirmation of complete imidazolide formation the reaction was cooled to 40-43°C under nitrogen and 4-amino-5-ethyl-1-methyl-1*H*-pyrazole-3-carboxamide (7.98 g, 47.5 mMol) charged in one portion before returning to reflux. A further 1 ml/g was distilled off at atmospheric pressure. The reaction was stirred at reflux for 6 hours. The reaction slurry was cooled and granulated at 10-15°C for one hour, filtered and washed with 1.5 mL/g (2% water in ethyl acetate). The solid was dried in vacuo overnight at 50°C to afford 21.2 g, 90.5% of a white crystalline solid. m.p. = 180°C. δ (CDCl₃): 1.04 (3H, t), 1.24 (3H, t), 1.59 (3H, t), 2.42 (2H, q), 2.54 (4H, t), 2.91 (2H, q), 3.12 (4H, t), 3.88 (3H, s), 4.79 (2H, q), 5.38 (1H, s), 6.67 (1H, s), 8.66 (1H, m), 8.86 (1H, m), 10.56 (1H, s). m/z = 493.2 [M+H]⁺, 100%, C₂₁H₃₁N₇O₅S requires 493.5.

### (6d) (R)-1-Ethyl-4-[3-(3-ethyl-6,7-dihydro-2-methyl-7-oxo-2H-pyrazolo[4,3-d]pyrimidin-5-yl)-2-(2-methoxy-1-methylethoxy)-5-pyridylsulfonyl]piperazine besylate salt (Compound IB).

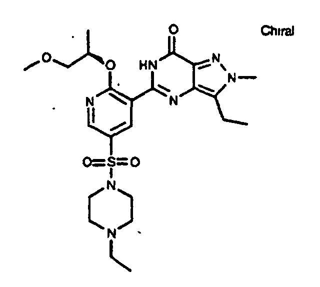

*N*-(3-Carbamoyl-5-ethyl-1-methyl-1*H*-pyrazol-4-yl)-2-ethoxy-5-(4-ethyl-1-piperazinylsulfonyl)nicotinamide (20 g, 40.5 mMol) and (*R*)-2-hydroxy-3-methoxy-propane (200 mL) were stirred in the reaction vessel under nitrogen at ambient temperature. Potassium *tert*-butoxide (10.9 g, 97.2 mMol) was added portion-wise over 2 min. Ethyl pivalate (15.4 mL, 0.1 Mol) was added and the reaction was heated to 120°C. Solvent was continuously distilled while further ethyl pivalate (0.3 Mol) and (*R*)-2-hydroxy-3-methoxy-propane was added to maintain the solvent volume at 200 mL. After approximately nine hours the reaction was cooled to ambient temperature and diluted with CH₂Cl₂ (200 mL). The pH was adjusted to 8 by slow addition of 6 M HCl (aq). To the resultant suspension was added water (200 mL) and the organic phase separated. The aqueous phase was extracted with CH₂Cl₂ (2 x 150 mL) and combined organic phases were transferred into a separate vessel. The volatile organic solvent was stripped and replaced with ethyl methyl ketone (200 mL) and then maintained at 50°C for 30 min. To this solution was added benzene sulphonic acid (7.7 g, 48.6 mMol) as a solution in ethyl methyl ketone (80 mL) dropwise. The reaction was stirred at 80°C for 90 min before ethyl methyl ketone (200 mL) was distilled. The resultant slurry was cooled to ambient temperature and granulated overnight. Filtration, washing with ice-cold ethyl methyl ketone (50 mL) and drying under vacuum afforded (*R*)-1-Ethyl-4-[3-(3-ethyl-6,7-dihydro-2-methyl-7-oxo-2*H*-pyrazolo[4,3-*d*]pyrimidin-5-yl)-2-(2-methoxy-1-methylethoxy)-5-pyridylsulfonyl]piperazine besylate salt, 24.0 g, 79% as a pale yellow solid. m.p. = 212-214°C. δ (CDCl₃): 1.16 (3H, t), 1.25 (3H, t), 1.31 (3H, d), 2.77 (2H, m), 2.93 (2H, m), 3.15 (4H, m), 3.25 (3H, s), 3.54 (4H, m), 3.82 (2H, d), 4.02 (3H, s), 5.44 (1H, m), 7.28 (3H, m), 7.57 (2H, m), 8.36 (1H, m), 8.71 (1H, m), 9.22 (1H, s, br), 11.57 (1H, s, br). m/z Found: 517.91 [M-H]⁺, (C₂₃H₃₃N₇O₅S requires 519.63, salt fragments to free base in MS).

Thus the yield of final product (IA) when using the hydroxide trapping agent is very good. Furthermore in accordance with a preferred embodiment of the invention a material suitable for clinical use can be provided directly.

Additionally, in accordance with the invention, the intermediate compounds VII and VI (more particularly VIIA, VIA and VIIB, VIB) can be prepared from commercially available starting materials (2-hydroxy nicotinic acid) in better yield than the corresponding reaction sequence in PCT/IB99/00519. For example, compound VII (wherein P and X are OEt) is formed in a yield of 14.5% in preparation 18 of PCT/IB99/00519 (i.e. from a reaction sequence of preparation 1,3,5,7 and 18) whereas the same compound is prepared in a yield of 23% in accordance with the present invention (see examples 1 g to 1 k). More preferably the whole or part of the reaction sequence for the formation of compounds VII and VI can be telescoped together in accordance with the invention to provide an even better yield . Thus compound VI (wherein X is OEt) is prepared in a yield of 35% (see example 4 herein). Furthermore, the reaction scheme of the present invention is safer and cheaper to operate, and in the case of the telescoped process also involves less steps (and processing time).

In a preferred aspect compounds of formula (I),(IA) and (IB) are prepared from nicotinic acid in accordance with Schemes 1 to 3.

Thus, in a preferred aspect of the invention there is provided a process for the preparation of a compound of formula (I), (IA) and (IB) which process is started by reacting 2-hydroxynicotinic acid or a salt thereof in the presence of SO₃ in a solvent to form a compound of formula (XI).

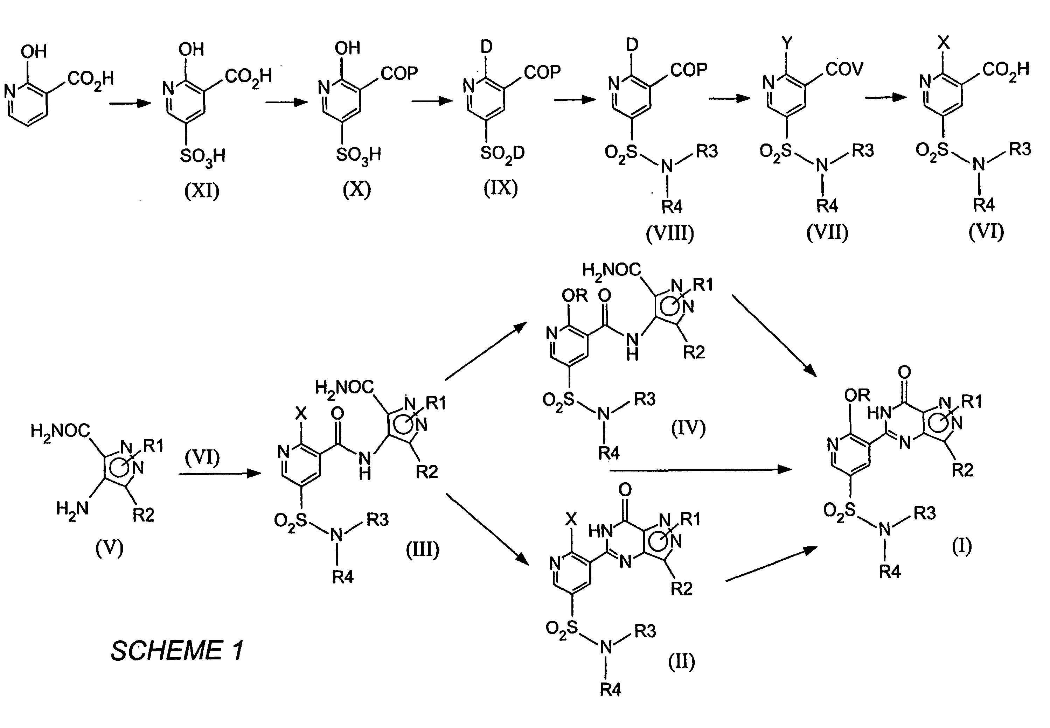

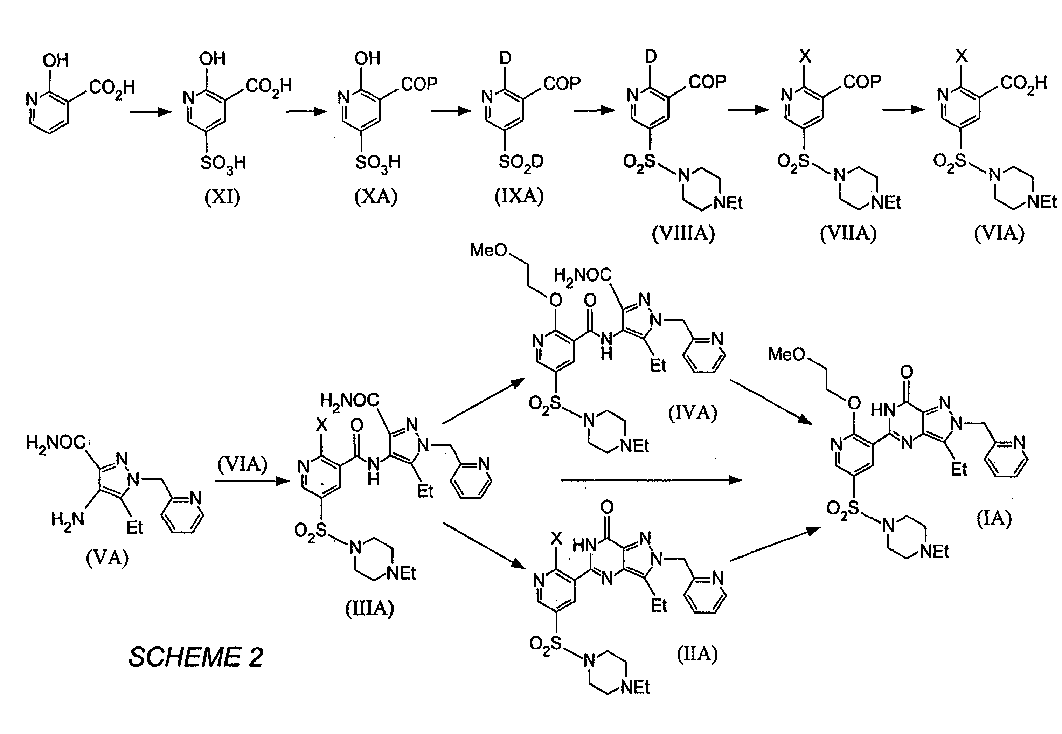

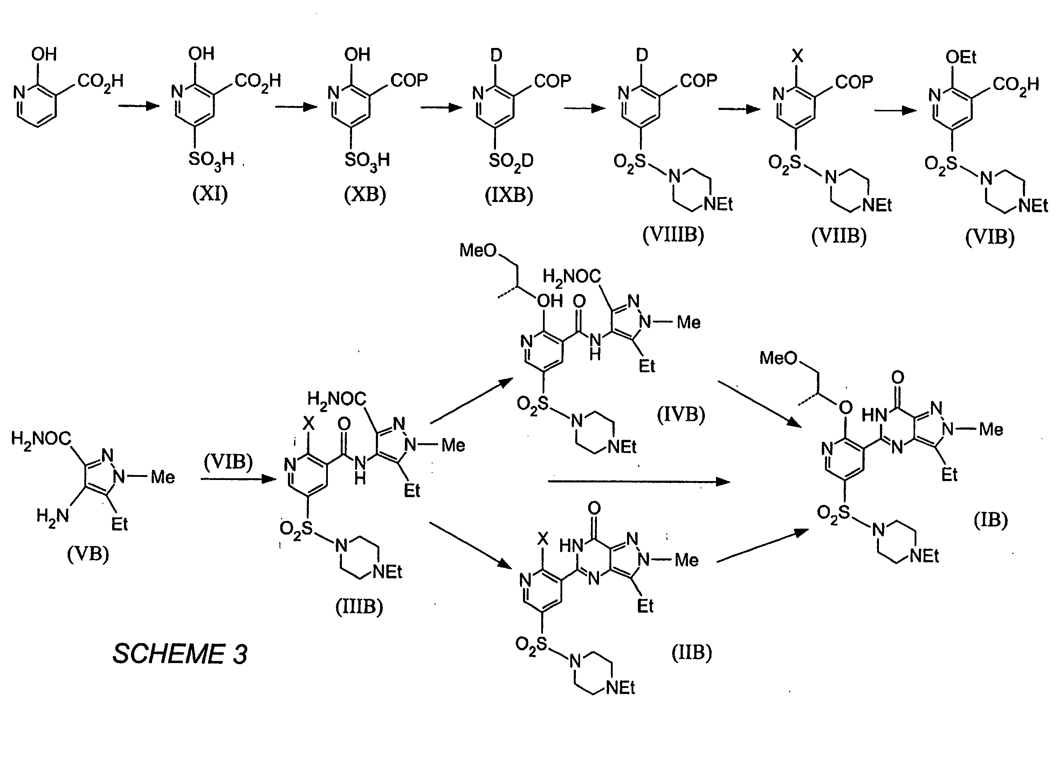

## Claims

1. A process for the preparation of a compound of formula (I) 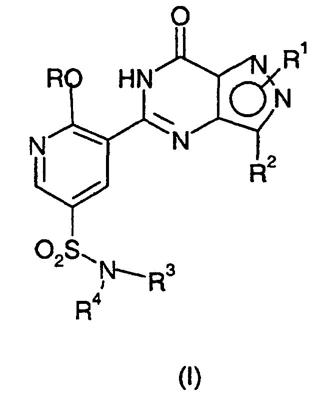 wherein
R is C₁ to C₆ alkyl optionally substituted with one or two substituents selected from C₃ to C₅ cycloalkyl, OH, C₁ to C₄ alkoxy, benzyloxy, NR⁵R⁶, phenyl, furanyl and pyridinyl; C₃ to C₆ cycloalkyl; 1-(C₁ to C₄ alkyl)piperidinyl; tetrahydrofuranyl or tetrahydropyranyl and wherein said C₁ to C₆ alkyl or said C₁ to C₄ alkoxy groups are optionally substituted by haloalkyl;
R¹ (which can be linked to either nitrogen of the pyrazole ring) is C₁ to C₃ alkyl, optionally substituted with phenyl, Het or a N linked heterocyclic group selected from piperidinyl and morpholinyl and wherein said phenyl group is optionally substituted by: C₁ to C₄ alkyl which is optionally substituted by haloalkyl or haloalkoxy; or C₁ to C₄ alkoxy; or halo or CN;
R² is C₁ to C₆ alkyl;
and Het is a C-linked 6-membered heterocyclic group containing one or two nitrogen atoms, optionally in the form of its mono-N-oxide, or a C-linked 5-membered heterocyclic group containing two or three nitrogen atoms, wherein
either of said heterocyclic groups is optionally substituted with C₁ to C₄ alkyl or C₁ to C₄ alkoxy or NHR⁷wherein R⁷ is H, C₁ to C₄ alkyl or C₁ to C₄ alkanoyl;
R³ and R⁴, together with the nitrogen atom to which they are attached, form a 4-R⁸-piperazinyl group optionally substituted with one or two C₁ to C₄ alkyl groups and optionally in the form of its 4-N-oxide;
R⁵ and R⁶ are each independently selected from Hand C₁ to C₄ alkyl optionally substituted with C₃ to C₅ cycloalkyl or C₁ to C₄ alkoxy, or, together with the nitrogen atom to which they are attached, form an azetidinyl, pyrrolidinyl, piperidinyl or morpholinyl group;
R⁸ is H; C₁ to C₄ alkyl optionally substituted with one or two substituents selected from OH, NR⁵R⁶, CONR⁵R⁶, phenyl optionally substituted with C₁ to C₄ alkoxy, benzodioxolyl and benzodioxanyl; C₃ to C₆ alkenyl; pyridinyl or pyrimidinyl;
said process comprising reacting a compound of formula (II), (III) or (IV) in the presence of ⁻OR and a hydroxide trapping agent wherein the compounds of formula II are formed from the compounds of formula III or in the case of compounds of formula (IV) reacting in the presence of an auxiliary base and a hydroxide trapping agent (i.e. ⁻OR is substituted by the auxiliary base) wherein X is a leaving group and R¹ to R⁴ are as defined above,

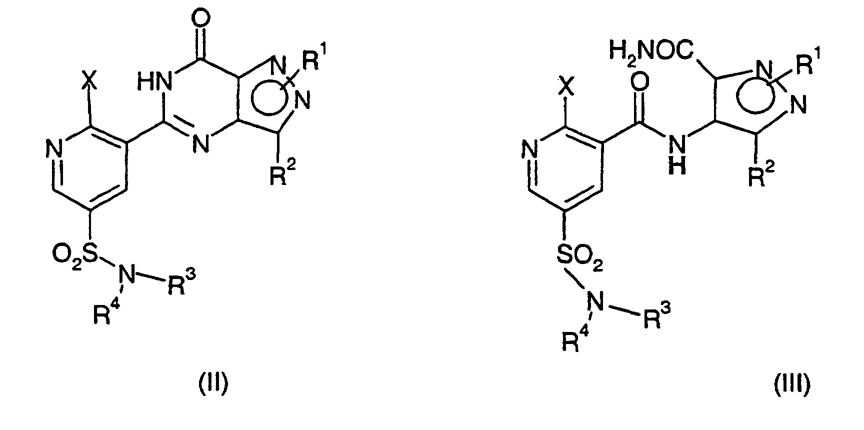

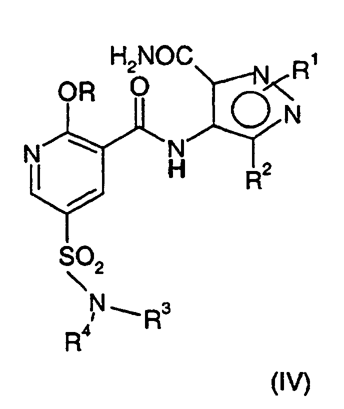

2. A process as claimed in claim 1 for the preparation of a compound of formulae (IA) and (IB) 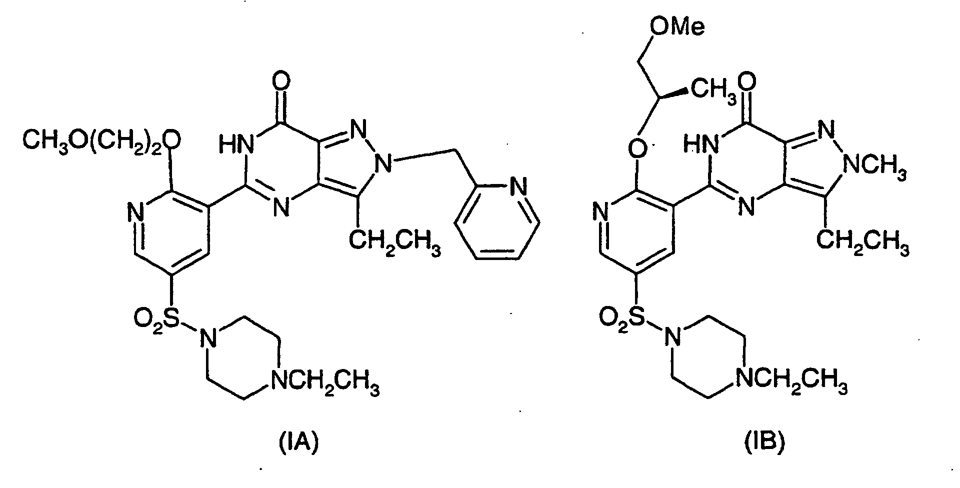 comprising reacting a compound of formula (IIA), (IIIA) or (IVA), and (IIB), (IIIB) or (IVB) respectively 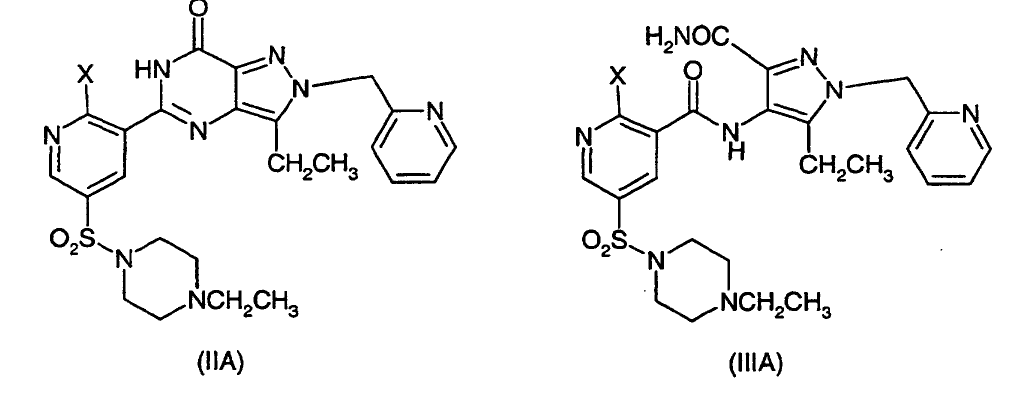 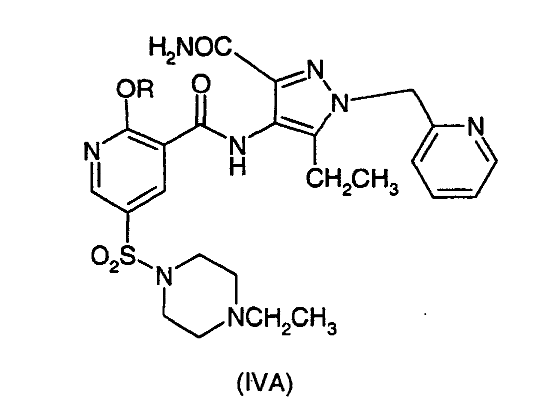 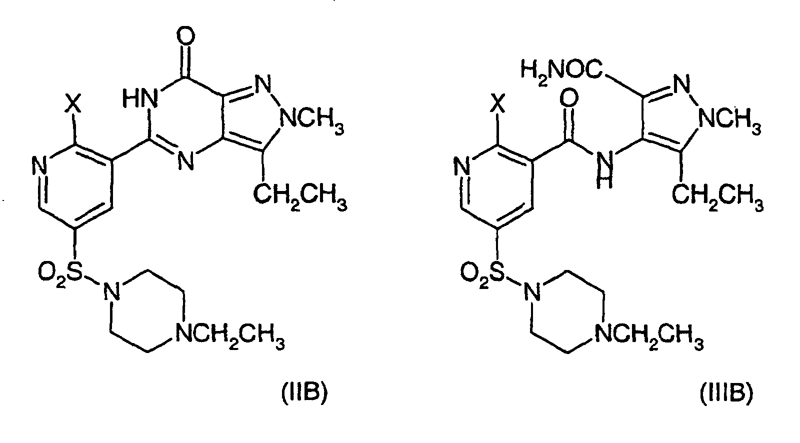 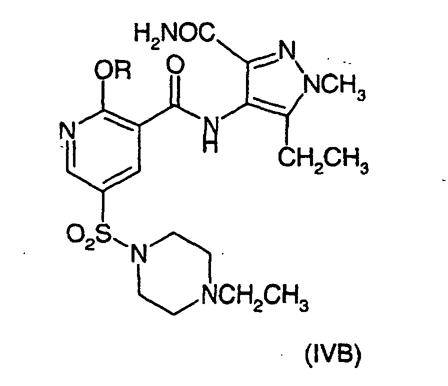 in the presence of ⁻OR and a hydroxide trapping agent, or alternatively in the case of compounds of formula (IVA) and (IVB) reacting in the presence of ⁻OR and an auxiliary base wherein OR in the case of formation of compound (IA) is CH₃O(CH₂)₂O-, and OR in the case of formation of compound (IB) is *(R)*-CH₃OCH₂CH(CH₃)O- and wherein X in formulae (IIA) to (IVA) and formulae (IIB) to (IVB) is a leaving group.

3. A process as claimed in claims 1 or 2 wherein the hydroxide trapping agent is an ester.

4. A process as claimed in any of claims 1 to 3 wherein the hydroxide trapping agent is an ester of the formula:
TOC(O)W
wherein OT is OR or the residue of a bulky alcohol or a non-nucleophilic alcohol or TOH is an alcohol which can be azeotropically removed during the reaction; and C(O)W is the residue of a carboxylic acid.

5. A process as claimed in any one of the preceding claims wherein X is selected from the group consisting of arylsulphonyloxy, C₁-C₄ alkylsulphonyloxy, nitro or halo substituted benzenesulphonyloxy, C₁-C₄ perfluoroalkylsulphonyloxy, optionally substituted aroyloxy, C₁-C₄ perfluoroalkanoyloxy, C₁-C₄ alkanoyloxy, halo; diazonium; C₁-C₄ primary and secondary alkoxy, oxonium, perchloryloxy, quatenaryammonium C₁-C₄ alkylsulphonuloxy, halosulphonyloxy, halonium and diarylsulphonylamino.

6. A process as claimed in claim 5 wherein X is a C₁₋₆ alkoxy.

7. A process as claimed in any one of the preceding claims wherein ⁻OR is present with an auxiliary base.

8. A process as claimed in claim 7 wherein the auxiliary base is selected from the group consisting of sterically hindered base , a metal hydride, metal oxide, metal carbonate and metal bicarbonate.

9. A process as claimed in claim 8 wherein the sterically hindered base is a metal salt of a sterically hindered alcohol or amine.

10. A process as claimed in claim 9 wherein the reaction is carried out in an inert solvent or ROH or a mixture of both.

11. A process as claimed in claim 10 wherein the solvent is selected from the group consisting of ROH , a secondary or tertiary C₄-C₁₂ alkanol, a C₃-C₁₂ cycloalkanol, a tertiary C₄-C₁₂ cycloalkanol, a secondary or tertiary (C₃-C₇ cycloalkyl)C₂-C₆ alkanol, a C₃-C₉ alkanone, 1,2-dimethoxyethane, 1,2-diethoxyethane, diglyme, tetrahydrofuran, 1,4-dioxan, toluene, xylene, chlorobenzene, 1,2-dichlorobenzene, acetonitrile, dimethyl sulphoxide, sulpholane, dimethylformamide, N-methylpyrrolidin-2-one, pyridine, and mixtures thereof.

12. A process as claimed in claim 11 wherein the solvent is ROH.

13. A process as claimed in any of claims 2 to 12 wherein compound (IA) is formed by reaction of compound (IIA) or (IIIA):
a) with 2-methoxyethanol and auxiliary base, optionally in an inert solvent and in the presence of said trapping agent; or
b) with ZO(CH₂)₂OCH₃ and an auxiliary base in 2-methoxyethanol or an inert solvent or both, in the presence of said trapping agent; or
c) with ZO(CH₂)₂OCH₃ and 2-methoxyethanol or an inert solvent or both, in the presence of said trapping agent, and wherein Z is a metal.

14. A process as claimed in any of claims 2 to 12 wherein compound (IB) is formed by reaction of compound (IIB) or (IIIB):
a) with (*R*) -CH₃OCH₂CH(CH₃)OH and auxiliary base, optionally in an inert solvent and in the presence of said trapping agent; or
b) with (*R*) -CH₃OCH₂CH(CH₃)OZ and an auxiliary base in (*R*) - CH₃OCH₂CH(CH₃)OH or an inert solvent or both, in the presence of said trapping agent; or)
c) with (*R*) -CH₃OCH₂CH(CH₃)OZ and (*R*) -CH₃OCH₂CH(CH₃)OH or an inert solvent or both, in the presence of said trapping agent, and wherein Z is a metal.

15. A process as claimed in any of the preceding claims wherein a compound of formula (III) is formed by coupling a compound of formula (V) with a compound of formula (VI) or a salt thereof.

16. A process according to claim 15 wherein a compound of formula (VI) or a salt thereof is formed from a compound of formula (VII) 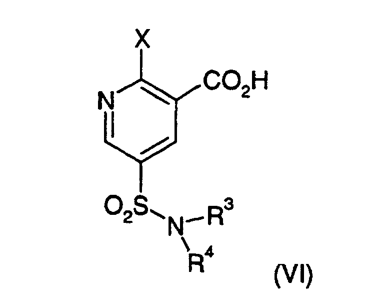 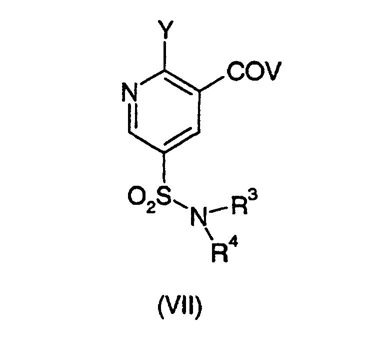
(a) for a compound formula (VI) wherein X is arylsulfonyloxy, C₁-C₄ alkylsulfonyloxy, C₁-C₄ perfluoroalkylsulfonyloxy, aryloxy, C₁-C₄ perfluoroalkanoyloxy, C₁-C₄ alkanoyloxy, quarternaryammonium C₁-C₄ alkylsulfonyloxy or halosulfonyloxy, comprising reacting a compound of formula (VII) wherein Y and V are OH in the presence of an appropriate sulphonylating, arylating or acylating agent of X;
(b) for a compound of formula (VI) wherein X is Cl, comprising reaction of a compound of formula (VII) wherein Y is Cl and V is P and P is a protecting group, with a deprotecting agent;
(c) for a compound of formula (VI) wherein X is diazonium, comprising reacting a compound of formula (VII) wherein Y is NH₂, V is OH with nitrous acid ;
(d) for a compound of formula (VI) wherein X is (diarylsulfonyl)amino comprising reacting a compound of formula (VII) wherein Y=NH₂ and V=OH in the presence of an appropriate sulphonylating agent;
(e) for a compound of formula (VI) wherein X is OR which is C₁₋₆ alkoxy, comprising reacting a compound of formula (VII) wherein V is P where P is a protecting group and Y is a C₁₋₆ primary or secondary alkoxy with a deprotecting agent.

17. A process for the preparation of compounds of formula (VII) as shown in claim 16 from compounds of formula (VIII) 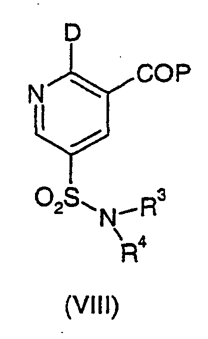 wherein D is Cl or Br and P is a protecting group,
(a) wherein for compounds of compound (VII) wherein Y is OH and V is OH, comprising reacting a compound of formula (VIII) with a hydrolising agent and optionally a further deprotecting agent where P is not deprotected by the hydrolysing agent;
(b) wherein for compounds of formula (VII) wherein Y is NH₂ and V is OH, comprising reacting a compound of formula (VIII) with an ammoniating agent to form an intermediate compound of formula (VII) wherein Y is NH₂ and V is P (a protecting group) and the reacting said intermediate (VII) with a deprotecting agent; and
(c) wherein for compounds of compound (VII) wherein Y is OR which is a C₁₋₆ alkoxy and V is P, comprising reacting a compound of formula (VIII) in the presence of ⁻OR;

18. A process for the preparation of a compound of formula (VIII) according to claim 17 comprising reaction of compounds of formula (IX) in the presence of an amine NH(R3)(R4) wherein R³ and R⁴ are as defined in claim 1, 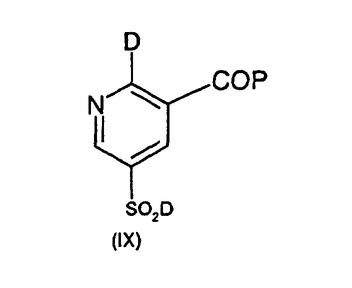 wherein D and P are as defined in claim 17.

19. A process for the preparation of a compound of formula (IX) according to claim 18 comprising reacting a compound of formula (X) with a chlorinating or brominating agent 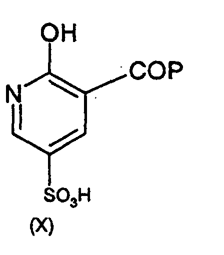 wherein P is as defined in claim 17.

20. A process according to claim 19 for preparation of a compound of formula (X) comprising reacting a compound of formula (XI) in the presence of an agent which will form a protecting group (P) on the carboxylic acid

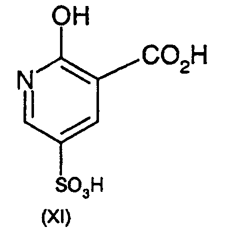

21. A process according to claim 20 for the preparation of a compound of formula (XI) comprising reacting 2-hydroxynicotinic acid or a salt thereof in the presence of SO₃ in a solvent.

22. A process according to claim 21 wherein the SO₃ is in an aprotic solvent , a mineral acid, or a liquid carboxylic acid.

23. A process according to any one of claims 16 to 22 wherein the compounds of formula (VI), (VII) and (VIII) are (VIA), (VIIA) and (VIIIA) respectively 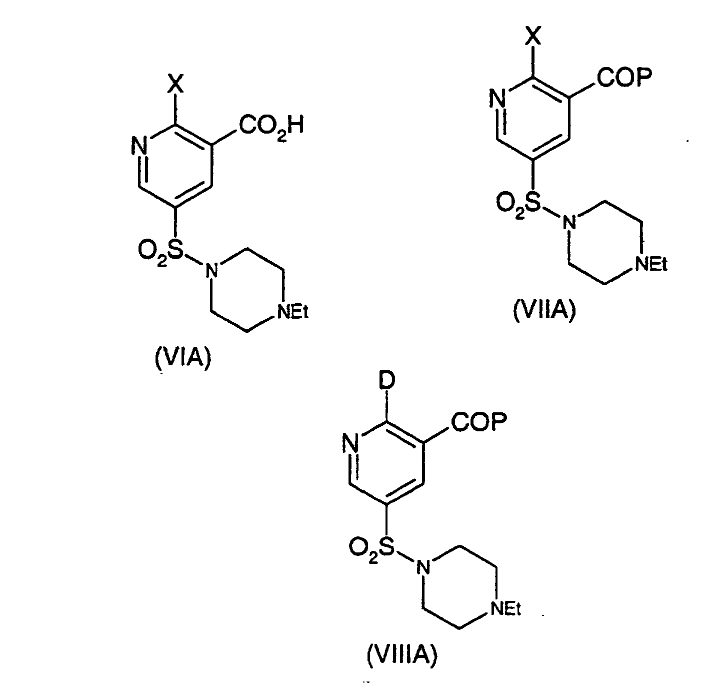 wherein X is a C₁₋₆ alkoxy, D and P are as defined in claims 16 and 17 , and the compounds (VIA) and (VIIA) are formed according to claim 16(e) and claim 17( c) respectively and compound (VIIIA) is formed according to claim 18 by reacting compound (IX) with N-ethyl piperazine or a salt thereof.

24. A process according to claim 23 wherein X is OEt and the compound (VIA) is formed by reaction of compound (VIIA) with a deprotecting agent , and compound (VIIA) is formed by reaction of compound (VIIIA) in the presence of OEt.

25. A process according to claims 23 or 24 wherein compound (X) is formed by reacting compound (XI) or a salt thereof with ethanol to form a protecting group OEt.

26. A process according to any one of claims 23 to 25 wherein two or more consecutive steps in the formation of (VIA), (VIIA) or (VIIIA) are carried out in toluene and telescoped together in a single telescoped step without intermediate isolation.

27. A process according to any one of the preceding claims for the preparation of a compound of formula (I), (IA) or (IB) comprising starting from commercially available 2-hydroxynicotinic acid or a salt thereof and reacting in the presence of SO₃ in a solvent to form a compound of formula (XI).

28. Compound (IVA): 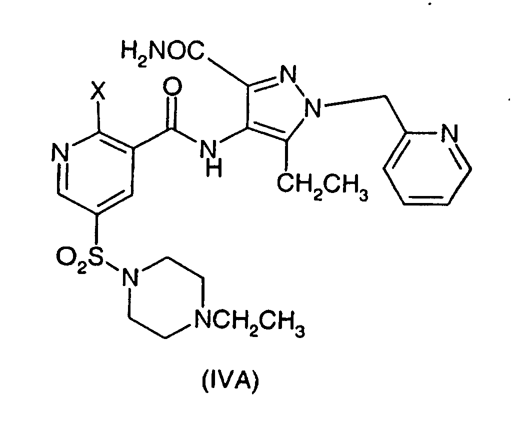 provided that X is not methoxyethoxy or ethoxy

29. Compound (IVB): 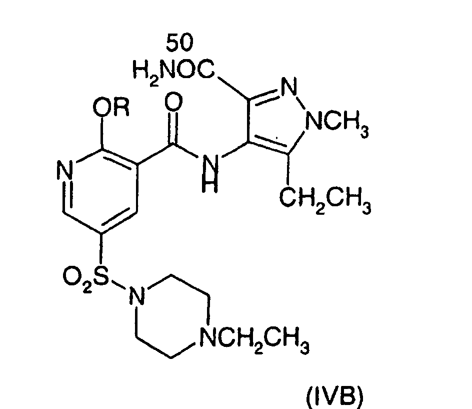 wherein R is defined in claim 1 provided that R is not ethyl.

## Patentansprüche

1. Verfahren zur Herstellung einer Verbindung der Formel (I) 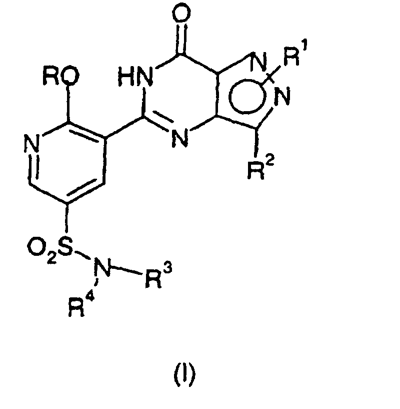 worin
R C₁ bis C₆-Alkyl, gegebenenfalls substituiert mit ein oder zwei Substituenten, ausgewählt aus C₃ bis C₅-Cycloalkyl, OH, C₁ bis C₄-Alkoxy, Benzyloxy, NR⁵R⁶, Phenyl, Furanyl und Pyridinyl; C₃ bis C₆-Cycloalkyl; 1-(C₁ bis C₄-Alkyl)piperidinyl; Tetrahydrofuranyl oder Tetrahydropyranyl ist, und worin die C₁ bis C₆-Alkyl- oder die C₁ bis C₄-Alkoarygruppen gegebenenfalls durch Halogenalkyl substituiert sind;
R¹ (das an einen der beiden Stickstoffe des Pyrazolringes gebunden sein kann) C₁ bis C₃-Alkyl, gegebenenfalls substituiert mit Phenyl, Het oder eine N-verknüpfte heterocyclische Gruppe, ausgewählt aus Piperidinyl und Morpholinyl, ist, und worin die Phenylgruppe gegebenenfalls durch: C₁ bis C₄-Alkyl, das gegebenenfalls durch Halogenalkyl oder Halogenalkoxy substituiert ist; oder C₁ bis C₄-Alkoxy oder Halogen oder CN substituiert ist;
R² C₁ bis C₆-Alkyl ist;
und
Het eine C-verknüpfte 6-gliedrige heterocyclische Gruppe, die ein oder zwei Stickstoffatome enthält, die gegebenenfalls in Form ihres Mono-N-Oxids vorliegen, oder eine C-verknüpfte 5-gliedrige heterocyclische Gruppe, die zwei oder drei Stickstoffatome enthält, ist, worin
eine der beiden heterocyclischen Gruppen gegebenenfalls mit C₁ bis C₄-Alkyl oder C₁ bis C₄-Alkoxy oder NHR⁷ substituiert ist, worin R⁷ H, C₁ bis C₄-Alkyl oder C₁ bis C₄-Alkanoyl ist; R³ und R⁴, zusammen mit dem Stickstoffatom, an das sie gebunden sind, eine
4-R⁸-Piperazinylgruppe bilden, die gegebenenfalls mit ein oder zwei C₁ bis C₄-Alkylgruppen substituiert ist, und gegebenenfalls in Form ihres 4-N-Oxids vorliegt;
R⁵ und R⁶ unabhängig voneinander aus H und C₁ bis C₄-Alkyl, gegebenenfalls substituiert mit C₃ bis C₅-Cycloalkyl, oder C₁ bis C₄-Alkoxy, ausgewählt sind, oder zusammen mit dem Stickstoffatom, an das sie gebunden sind, eine Azetidinyl, Pyrrolidinyl, Piperidinyl oder Morpholinylgruppe bilden;
R⁸ H, C₁ bis C₄-Alkyl, gegebenenfalls substituiert mit ein oder zwei Substituenten, ausgewählt aus OH, NR⁵R⁶, CONR⁵R⁶, Phenyl, gegebenenfalls substituiert mit C₁ bis C₄-Alkoxy, Benzodioxolyl und Benzodioxanyl; C₃ bis C₆-Alkenyl; Pyridinyl oder Pyrimidinyl ist;
wobei das Verfahren die Umsetzung einer Verbindung der Formel (II), (III) oder (IV) in Gegenwart von ⁻OR und einem Hydroxidabfangmittel, wobei die Verbindungen der Formel II aus den Verbindungen der Formel III gebildet werden, oder im Falle der Verbindungen der Formel (IV), die Umsetzung in Gegenwart einer Hilfsbase und eines Hydroxidabfangmittels (das heißt ⁻OR ist durch die Hilfsbase substituiert) umfaßt, wobei X eine Abgangsgruppe ist und R¹ bis R⁴ wie oben definiert sind,

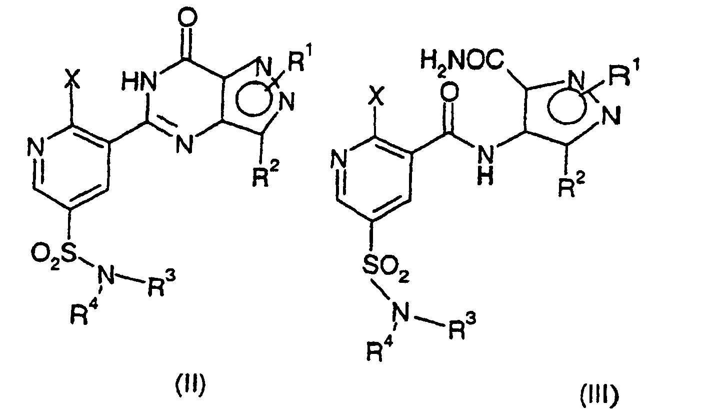

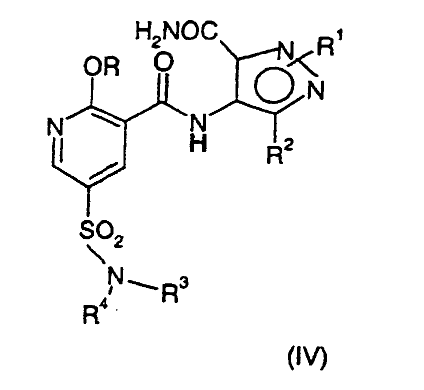

2. Verfahren nach Anspruch 1 zur Herstellung einer Verbindung der Formeln (IA) und (IB) 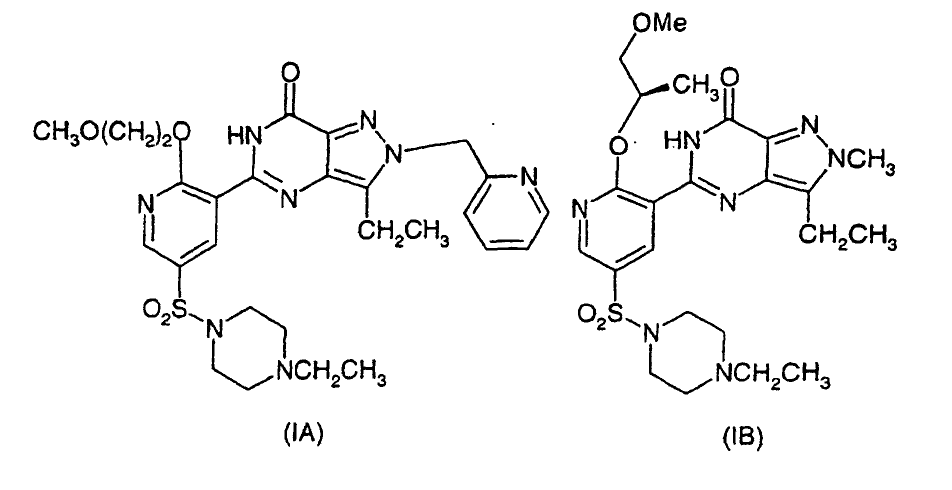 umfassend die Umsetzung einer Verbindung der Formel (IIA), (IIIA) oder (IVA), bzw. (IIB), (IIIB) oder (IVB) 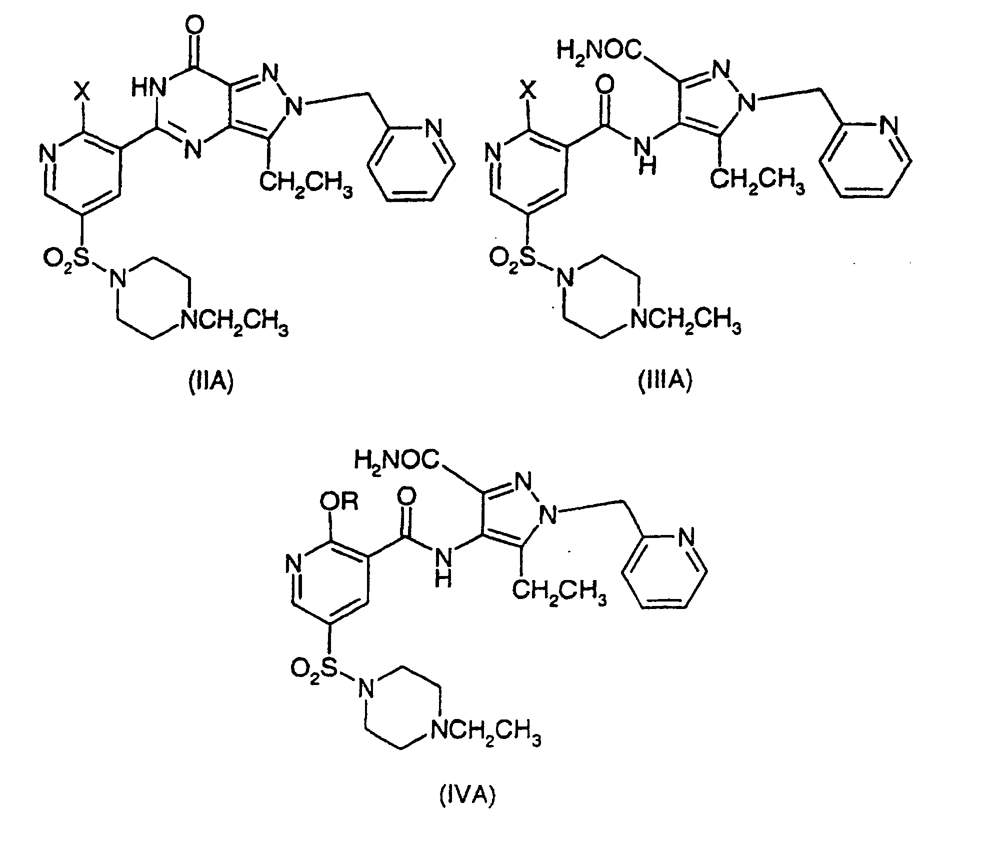 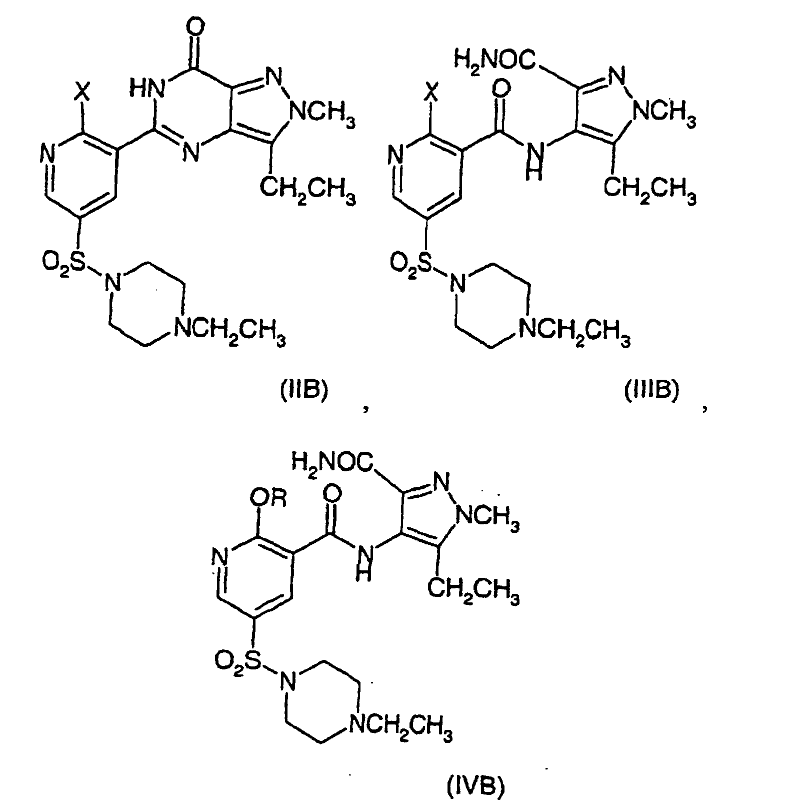 in Gegenwart von ⁻OR und eines Hydroxidabfangmittels oder alternativ im Falle von Verbindungen der Formel (IVA) und (IVB) die Umsetzung in der Gegenwart von ⁻OR und einer Hilfsbase, wobei OR im Falle der Bildung der Verbindung (IA) CH₃O(CH₂)₂O- ist und OR im Falle der Bildung der Verbindung (IB) (*R*)-CH₃OCH₂CH(CH₃)O- ist, und worin X in den Formeln (IIA) bis (IVA) und den Formeln (IIB) bis (IVB) eine Abgangsgruppe ist.

3. Verfahren nach den Ansprüchen 1 oder 2, wobei das Hydroxidabfangmittel ein Ester ist.

4. Verfahren nach einem der Ansprüche 1 bis 3, wobei das Hydroxidabfangmittel ein Ester der Formel:
TOC(O)W
ist, worin OT OR oder der Rest eines großen Alkohols oder eines nicht-nukleophilen Alkohols ist, oder TOH ein Alkohol ist, der während der Reaktion azeotrop entfernt werden kann; und C(O)W der Rest einer Carbonsäure ist.

5. Verfahren nach einem der vorherigen Ansprüche, wobei X aus der Gruppe, bestehend aus Arylsulfonyloxy, C₁-C₄-Alkylsulfonyloxy, Nitro- oder Halogen-substituiertem Benzensulfonyloxy, C₁-C₄-Perfluoralkylsulfonyloxy, gegebenenfalls substituiertem Aroyloxy, C₁-C₄-Perfluoralkanoyloxy, C₁-C₄-Alkanoyloxy, Halogen; Diazonium; primärem und sekundärem C₁-C₄-Alkoxy, Oxonium, Perchloryloxy, Quartärammonium-C₁-C₄-Alkylsulfonyloxy, Halogensulfonyloxy, Halonium und Diarylsulfonylamino ausgewählt ist.

6. Verfahren nach Anspruch 5, wobei X ein C₁-₆-Alkoxy ist.

7. Verfahren nach einem der vorherigen Ansprüche, wobei ⁻OR mit einer Hilfsbase vorliegt.

8. Verfahren nach Anspruch 7, wobei die Hilfsbase aus der Gruppe, bestehend aus sterisch gehinderter Base, einem Metallhydrid, Metalloxid, Metallcarbonat und Metallbicarbonat ausgewählt ist.

9. Verfahren nach Anspruch 8, wobei die sterisch gehinderte Base ein Metallsalz eines sterisch gehinderten Alkohols oder Amins ist.

10. Verfahren nach Anspruch 9, wobei die Reaktion in einem inerten Lösungsmittel oder ROH oder einem Gemisch aus beiden durchgeführt wird.

11. Verfahren nach Anspruch 10, wobei das Lösungsmittel aus der Gruppe, bestehend aus ROH, einem sekundären oder tertiären C₄-C₁₂-Alkanol, einem C₃-C₁₂-Cycloalkanol, einem tertiären C₄-C₁₂-Cycloalkanol, einem sekundären oder tertiären (C₃-C₇-Cycloalkyl)C₂-C₆ Alkanol, einem C₃-C₉-Alkanon, 1,2-Dimethoxyethan, 1,2-Diethoxyethan, Diethylenglykoldimethylether, Tetrahydrofuran, 1,4-Dioxan, Toluen, Xylen, Chlorbenzen, 1,2-Dichlorbenzen, Acetonitril, Dimethylsulphoxid, Sulpholan, Dimethylformamid, N-Methylpyrrolidin-2-on, Pyridin, und Gemischen hiervon ausgewählt ist.

12. Verfahren nach Anspruch 11, wobei das Lösungsmittel ROH ist.

13. Verfahren nach einem der Ansprüche 2 bis 12, wobei die Verbindung (IA) durch die Umsetzung der Verbindung (IIA) oder (IIIA):
a) mit 2-Methoxyethanol und einer Hilfsbase, gegebenenfalls in einem inerten Lösungsmittel, und in der Gegenwart des Abfangmittels; oder
b) mit ZO(CH₂)₂OCH₃ und einer Hilfsbase in 2-Methoxyethanol oder einem inerten Lösungsmittel oder beidem, in der Gegenwart des Abfangmittels, oder
c) mit ZO(CH₂)₂OCH₃ und 2-Methoxyethanol oder einem inerten Lösungsmittel oder beidem, in der Gegenwart des Abfangmittels, und wobei Z ein Metall ist,
gebildet wird.

14. Verfahren nach einem der Ansprüche 2 bis 12, wobei die Verbindung (IB) durch die Umsetzung der Verbindung (IIB) oder (IIIB):
a) mit (*R*)-CH₃OCH₂CH(CH₃)OH und einer Hilfsbase, gegebenenfalls in einem inerten Lösungsmittel, und in der Gegenwart des Abfangmittels, oder
b) mit (*R*)-CH₃OCH₂CH(CH₃)OZ und einer Hilfsbase in (*R*)-CH₃OCH₂CH(CH₃)OH oder einem inerten Lösungsmittel oder beidem, in der Gegenwart des Abfangmittels, oder
c) mit (*R*)-CH₃OCH₂CH(CH₃)OZ und (*R*)-CH₃OCH₂CH(CH₃)OH oder einem inerten Lösungsmittel oder beidem, in der Gegenwart des Abfangmittels, und wobei Z ein Metall ist,
gebildet wird.

15. Verfahren nach einem der vorherigen Ansprüche, wobei eine Verbindung der Formel (III) durch die Kopplung einer Verbindung der Formel (V) mit einer Verbindung der Formel (VI) oder einem Salz hiervon gebildet wird.

16. Verfahren nach Anspruch 15, wobei eine Verbindung der Formel (VI) oder ein Salz hiervon aus einer Verbindung der Formel (VII) gebildet wird, 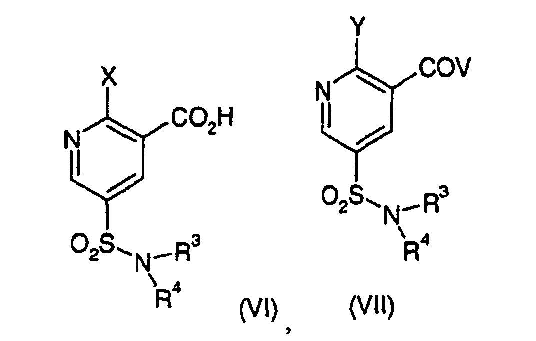
(a) für eine Verbindung der Formel (VI), worin X Arylsulfonyloxy, C₁-C₄-Alkylsulfonyloxy, C₁-C₄-Perfluoralkylsulfonyloxy, Aryloxy, C₁-C₄-Perfluoralkanoyloxy, C₁-C₄-Alkanoyloxy, Quartärammonium-C₁-C₄-Alkylsulfonyloxy oder Halogensulfonyloxy ist, durch die Umsetzung einer Verbindung der Formel (VII), worin Y und V OH sind, in der Gegenwart eines geeigneten Sulfonylierungs-, Arylierungs- oder Acylierungsmittels von X;
(b) für eine Verbindung der Formel (VI), worin X Cl ist, durch die Umsetzung einer Verbindung der Formel (VII), worin Y Cl ist und V P ist und P eine Schutzgruppe ist, mit einem Entschützungsmittel;
(c) für eine Verbindung der Formel (VI), worin X Diazonium ist, durch die Umsetzung einer Verbindung der Formel (VII), worin Y NH₂ ist, V OH ist, mit salpetriger Säure;
(d) für eine Verbindung der Formel (VI), worin X (Diarylsulfonyl)amino ist, durch die Umsetzung einer Verbindung der Formel (VII), worin Y = NH₂ und V = OH, in der Gegenwart eines geeigneten Sulfonylierungsmittels;
(e) für eine Verbindung der Formel (VI), worin X OR ist, das C₁-₆-Alkoxy ist, durch die Umsetzung einer Verbindung der Formel (VII), worin V P ist, wobei P eine Schutzgruppe ist, und Y ein primäres oder sekundäres C₁-₆-Alkoxy ist, mit einem Entschützungsmittel.

17. Verfahren zur Herstellung von Verbindungen der Formel (VII), wie in Anspruch 16 gezeigt, aus den Verbindungen der Formel (VIII), 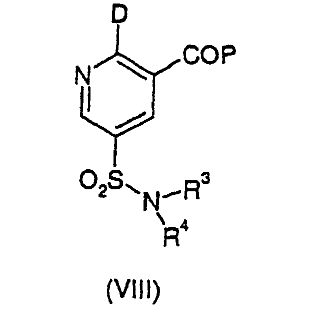 worin D Cl oder Br ist und P eine Schutzgruppe ist,
(a) umfassend für Verbindungen der Formel (VII), worin Y OH ist und V OH ist, die Umsetzung einer Verbindung der Formel (VIII) mit einem Hydrolysemittel und gegebenenfalls einem weiteren Entschützungsmittel, wobei P durch das Hydrolysemittel nicht entschützt wird;
(b) umfassend für Verbindungen der Formel (VII), worin Y NH₂ ist und V OH ist, die Umsetzung einer Verbindung der Formel (VIII) mit einem Ammonisierungsmittel, um eine Zwischenverbindung der Formel (VII) zu bilden, worin Y NH₂ und V P ist (eine Schutzgruppe) und die Umsetzung des Zwischenproduktes (VII) mit einem Entschützungsmittel; und
(c) umfassend für Verbindungen der Formel (VII), worin Y OR ist, das ein C₁-₆-Alkoxy ist, und V P ist, die Umsetzung einer Verbindung der Formel (VIII) in Gegenwart von OR.

18. Verfahren zur Herstellung einer Verbindung der Formel (VIII) gemäß Anspruch 17, umfassend die Umsetzung von Verbindungen der Formel (IX) in der Gegenwart eines Amins NH(R³)(R⁴) worin R³ und R⁴ wie in Anspruch 1 definiert sind, 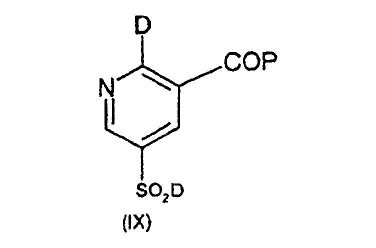 worin D und P wie in Anspruch 17 definiert sind.

19. Verfahren zur Herstellung einer Verbindung der Formel (IX) gemäß Anspruch 18, umfassend die Umsetzung einer Verbindung der Formel (X) mit einem Chlorierungs- oder Bromierungsmittel, 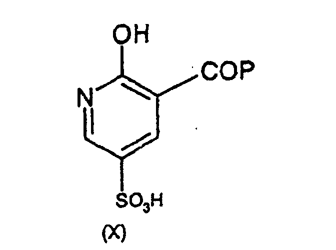 worin P wie in Anspruch 17 definiert ist

20. Verfahren nach Anspruch 19 zur Herstellung einer Verbindung der Formel (X), umfassend die Umsetzung einer Verbindung der Formel (XI) in der Gegenwart eines Mittels, das eine Schutzgruppe P an der Carbonsäure bilden wird

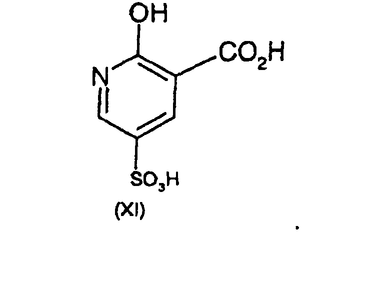

21. Verfahren nach Anspruch 20 zur Herstellung einer Verbindung der Formel (XI), umfassend die Umsetzung von 2-Hydroxynicotinsäure oder eines Salzes hiervon in der Gegenwart von SO₃ in einem Lösungsmittel.

22. Verfahren nach Anspruch 21, wobei das SO₃ in einem aprotischen Lösungsmittel, einer Mineralsäure oder einer flüssigen Carbonsäure vorliegt.

23. Verfahren nach einem der Ansprüche 16 bis 22, wobei die Verbindungen der Formel (VI), (VII) und (VIII) (VIA), (VIIA) bzw. (VIIIA) sind 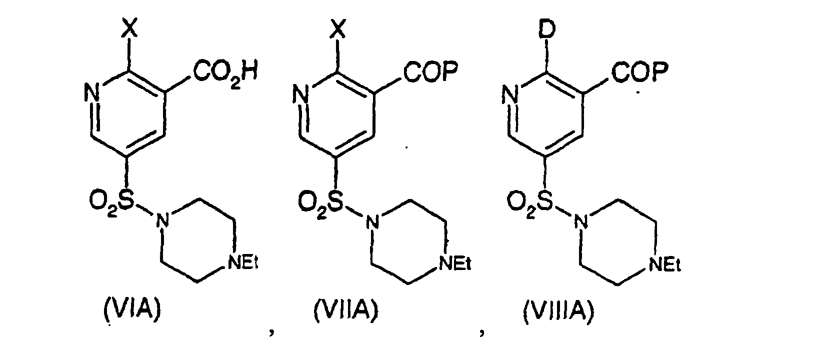 worin X ein C₁-₆-Alkoxy ist, D und P wie in den Ansprüchen 16 und 17 definiert sind, und die Verbindungen (VIA) und (VIIA) gemäß Anspruch 16(e) bzw. Anspruch 17(c) gebildet werden, und Verbindung (VIIIA) gemäß Anspruch 18 durch die Umsetzung der Verbindung (IX) mit N-Ethylpiperazin oder einem Salz hiervon gebildet wird.

24. Verfahren nach Anspruch 23, worin X OEt ist und die Verbindung (VIA) durch die Umsetzung der Verbindung (VIIA) mit einem Entschützungsmittel gebildet wird, und die Verbindung (VIIA) durch die Umsetzung der Verbindung (VIIIA) in Gegenwart von OEt gebildet wird.

25. Verfahren nach Anspruch 23 oder 24, wobei die Verbindung (X) durch die Umsetzung der Verbindung (XI) oder eines Salzes hiervon mit Ethanol gebildet wird, um eine Schutzgruppe OEt zu bilden.

26. Verfahren nach einem der Ansprüche 23 bis 25, wobei zwei oder mehr aufeinanderfolgende Schritte bei der Bildung von (VIA), (VIIA) oder (VIIIA) in Toluen durchgeführt werden und zu einem einzelnen zusammengeschobenen Schritt zusammengeschoben werden, ohne Zwischenisolierung.

27. Verfahren nach einem der vorherigen Ansprüche zur Herstellung einer Verbindung der Formel (I), (IA) oder (IB), umfassend den Ausgang von kommerziell erhältlicher 2-Hydroxynicotinsäure oder einem Salz hiervon und die Umsetzung in der Gegenwart von SO₃ in einem Lösungsmittel, um eine Verbindung der Formel (XI) zu bilden.

28. Verbindung (IVA): 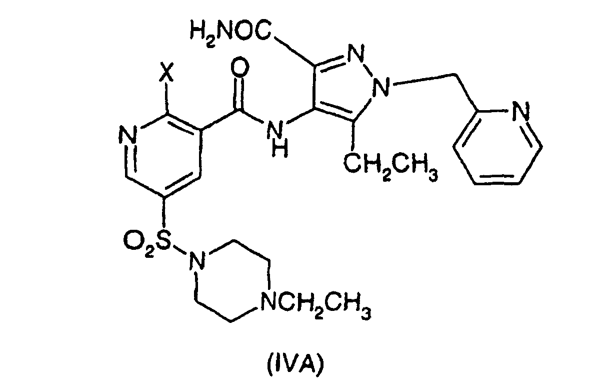 vorausgesetzt, daß X nicht Methoxyethoxy oder Ethoxy ist.

29. Verbindung (IVB): 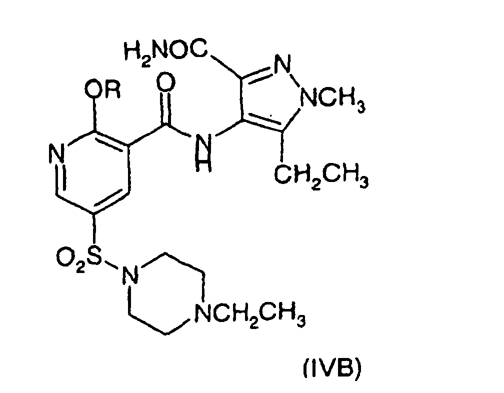 worin R wie in Anspruch 1 definiert ist, vorausgesetzt, daß R nicht Ethyl ist.

## Revendications

1. Procédé pour la préparation d'un composé de formule (I) 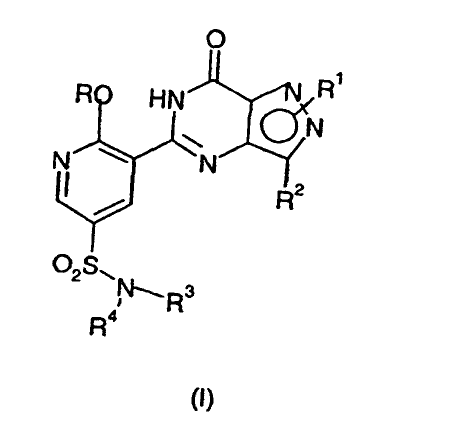 dans laquelle
R représente un groupe alkyle en C₁ à C₆ facultativement substitué avec un ou deux substituants choisis entre des substituants cycloalkyle en C₃ à C₅, OH, alkoxy en C₁ à C₄, benzyloxy, NR⁵R⁶, phényle, furannyle et pyridinyle ; cycloalkyle en C₃ à C₆ ; 1-(alkyle en C₁ à C₄)pipéridinyle ; tétrahydrofurannyle ou tétrahydropyrannyle, et dans laquelle lesdits groupes alkyle en C₁ à C₆ ou lesdits groupes alkoxy en C₁ à C₄ sont facultativement substitués avec des substituants halogénalkyle ;
R¹ (qui peut être lié à l'un ou l'autre atome d'azote du noyau pyrazole) représente un groupe alkyle en C₁ à C₃, facultativement substitué avec un groupe phényle, Het ou un groupe hétérocyclique, lié à N, choisi entre les groupes pipéridinyle et morpholinyle, ledit groupe phényle étant facultativement substitué avec : un groupe alkyle en C₁ à C₄ qui est facultativement substitué avec un substituant halogénalkyle ou halogénalkoxy ; ou un groupe alkoxy en C₁ à C₄ ; ou un groupe halogéno ou CN ;
R² représente un groupe alkyle en C₁ à C₆ ;
et
Het représente un groupe hétérocyclique hexagonal, lié à C, contenant un ou deux atomes d'azote, éventuellement sous forme de son mono-N-oxyde, ou un groupe hétérocyclique pentagonal, lié à C, contenant deux ou trois atomes d'azote,
et dans laquelle chacun desdits groupes hétérocycliques est facultativement substitué avec un substituant alkyle en C₁ à C₄ ou alkoxy en C₁ à C₄ ou NHR⁷ dans lequel R⁷ représente H, un groupe alkyle en C₁ à C₄ ou alkanoyle en C₁ à C₄ ;
R³ et R⁴, conjointement avec l'atome d'azote auquel ils sont fixés, forment un groupe 4-R⁸-pipérazinyle facultativement substitué avec un ou deux groupes alkyle en C₁ à C₄ et facultativement sous forme de son 4-N-oxyde ;
R⁵ et R⁶ sont choisis chacun indépendamment entre H et un groupe alkyle en C₁ à C₄ facultativement substitué avec un substituant cycloalkyle en C₃ à C₅ ou alkoxy en C₁ à C₄ ou bien, conjointement avec l'atome d'azote auquel ils sont fixés, forment un groupe azétidinyle, pyrrolidinyle, pipéridinyle ou morpholinyle ;
R⁸ représente H ; un groupe alkyle en C₁ à C₄ facultativement substitué avec un ou deux substituants choisis entre des substituants OH, NR⁵R⁶, CONR⁵R⁶, phényle facultativement substitué avec un substituant alkoxy en C₁ à C₄, benzodioxolyle et benzodioxanyle ; alcényle en C₃ à C₆ ; pyridinyle ou pyrimidinyle ;
ledit procédé comprenant la réaction d'un composé de formules (II), (III) ou (IV) en présence de ⁻OR et d'un agent de piégeage d'hydroxyde, les composés de formule (II) étant formés à partir des composés de formule (III) ou, dans le cas de composés de formule (IV), une réaction en présence d'une base auxiliaire et d'un agent de piégeage d'hydroxyde (c'est-à-dire que ⁻OR est remplacé par la base auxiliaire) où X représente un groupe partant et R¹ à R⁴ répondent aux définitions précitées,

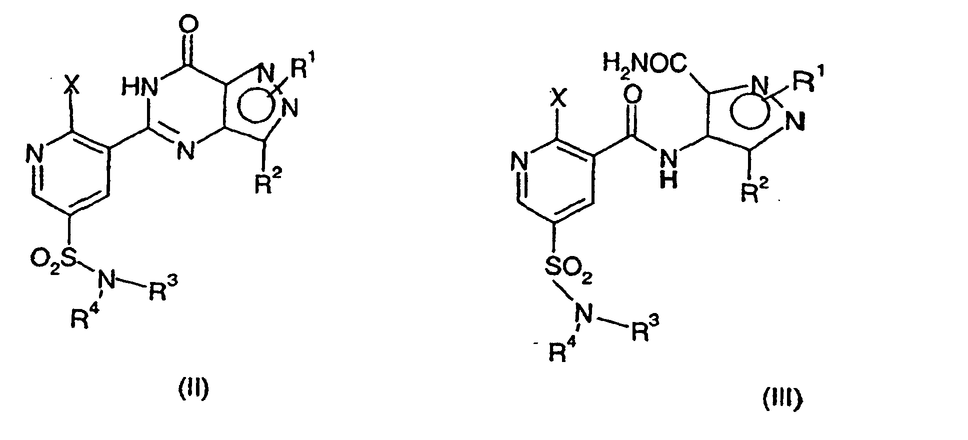

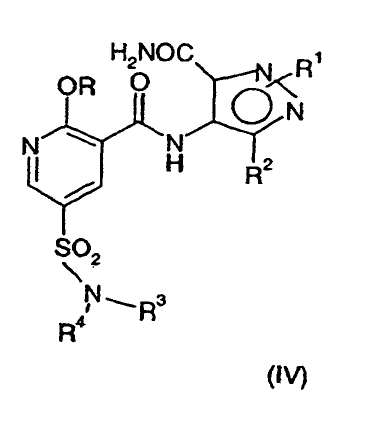

2. Procédé suivant la revendication 1 pour la préparation d'un composé de formule (IA) ou (IB) 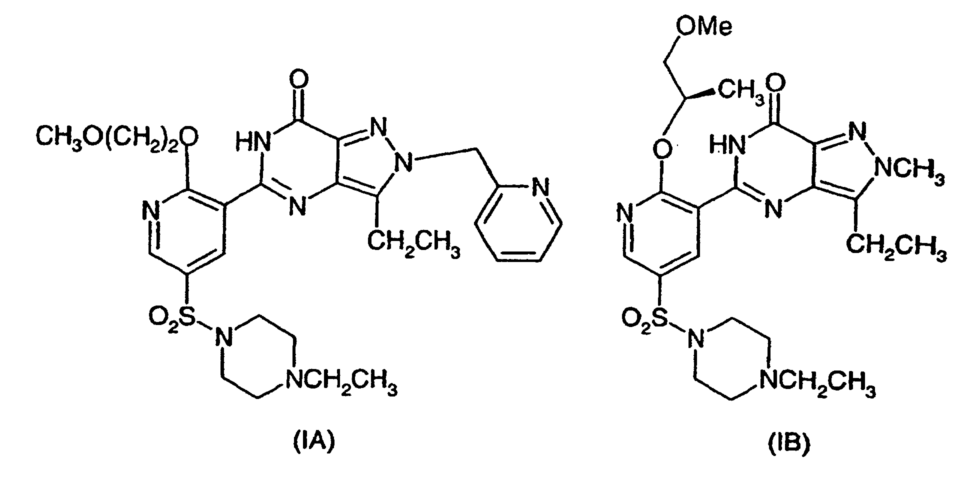 comprenant la réaction d'un composé de formules (IIA), (IIIA) et (IVA) et d'un composé de formules (IIB), (IIIB) ou (IVB), respectivement 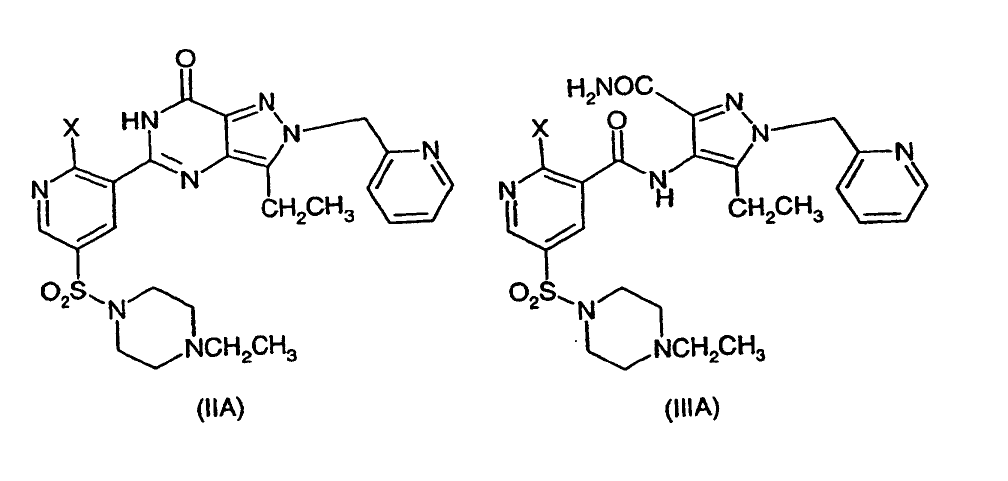 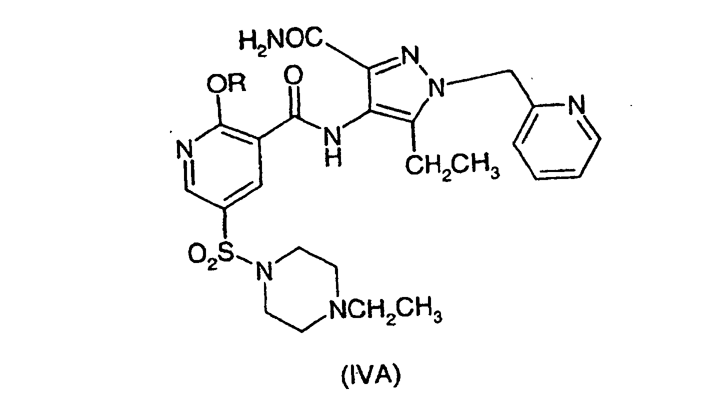 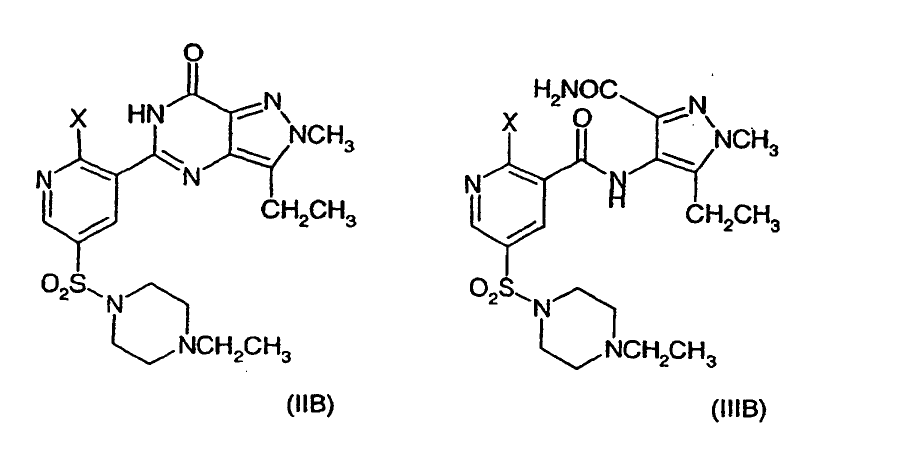 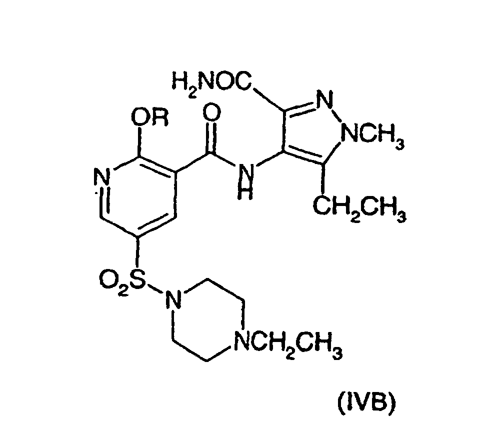 en présence de ⁻OR et d'un agent de piégeage d'hydroxyde ou, en variante, dans le cas de composés de formules (IVA) et (IVB), la réaction en présence de ⁻OR et d'une base auxiliaire, le groupe OR dans le cas de la formation du composé (IA) représente un groupe CH₃O(CH₂)₂O-, et le groupe OR dans le cas de la formation du composé (IB) représente un groupe *(R)*-CH₃OCH₂CH(CH₃)O- et X dans les formules (IIA) à (IVA) et les formules (IIB) à (IVB) représente un groupe partant.

3. Procédé suivant la revendication 1 ou 2, dans lequel l'agent de piégeage d'hydroxyde est un ester.

4. Procédé suivant l'une quelconque des revendications 1 à 3, dans lequel l'agent de piégeage d'hydroxyde est un ester de formule :
TOC(O)W
dans laquelle OT représente un groupe OR ou le résidu d'un alcool volumineux ou d'un alcool non nucléophile, ou bien TOH représente un alcool qui peut être éliminé de manière azéotrope au cours de la réaction ; et C(O)W représente le résidu d'un acide carboxylique.

5. Procédé suivant l'une quelconque des revendications précédentes, dans lequel X est choisi dans le groupe consistant en des groupes arylsulfonyloxy, alkylsulfonyloxy en C₁ à C₄, benzènesulfonyloxy à substituant nitro ou halogéno, perfluoralkylsulfonyloxy en C₁ à C₄, aroyloxy facultativement substitué, perfluoralcanoyloxy en C₁ à C₄, alcanoyloxy en C₁ à C₄, halogéno ; diazonium ; alkoxy en C₁ à C₄ primaire ou secondaire, oxonium, perchloryloxy, ammonium quaternaire(alkyle en C₁ à C₄)sulfonyloxy, halogénosulfonyloxy, halonium et diarylsulfonylamino.

6. Procédé suivant la revendication 5, dans lequel X représente un groupe alkoxy en C₁ à C₆.

7. Procédé suivant l'une quelconque des revendications précédentes, dans lequel ⁻OR est présent avec une base auxiliaire.

8. Procédé suivant la revendication 7, dans lequel la base auxiliaire est choisie dans le groupe consistant en une base à encombrement stérique, un hydrure métallique, un oxyde métallique, un carbonate métallique et un bicarbonate métallique.

9. Procédé suivant la revendication 8, dans lequel la base à encombrement stérique est un sel métallique d'un alcool ou d'une amine à encombrement stérique.

10. Procédé suivant la revendication 9, dans lequel la réaction est conduite dans un solvant inerte ou ROH ou un de leurs mélanges.

11. Procédé suivant la revendication 10, dans lequel le solvant est choisi dans le groupe consistant en ROH, un alcanol secondaire ou tertiaire en C₄ à C₁₂, un cycloalcanol en C₃ à C₁₂, un cycloalcanol tertiaire en C₄ à C₁₂, un (cycloalkyle en C₃ à C₇) (alcanol en C₂ à C₆) secondaire ou tertiaire, un alcanone en C₃ à C₉, le 1,2-diméthoxyéthane, le 1,2-diéthoxyéthane, le diglyme, le tétrahydrofuranne, le 1,4-dioxanne, le toluène, le xylène, le chlorobenzène, le 1,2-dichlorobenzène, l'acétonitrile, le diméthylsulfoxyde, le sulfolane, le diméthylformamide, la N-méthylpyrrolidine-2-one, la pyridine et leurs mélanges.

12. Procédé suivant la revendication 11, dans lequel le solvant consiste en ROH.

13. Procédé suivant l'une quelconque des revendications 2 à 12, dans lequel le composé (IA) est formé par réaction d'un composé (IIA) ou (IIIA) :
a) avec du 2-méthoxyéthanol et une base auxiliaire, facultativement dans un solvant inerte et en présence dudit agent de piégeage ; ou
b) avec un composé de formule ZO(CH₂)₂OCH₃ et une base auxiliaire dans du 2-méthoxyéthanol ou un solvant inerte ou bien à la fois du 2-méthoxyéthanol et ledit solvant, en présence dudit agent de piégeage ; ou
c) avec un composé de formule ZO(CH₂)₂OCH₃ et du 2-méthoxyéthanol ou un solvant inerte ou bien à la fois du 2-méthoxyéthanol et ledit solvant, en présence dudit agent de piégeage, Z représentant un métal.

14. Procédé suivant l'une quelconque des revendications 2 à 12, dans le composé (IB) est formé par réaction du composé (IIB) ou (IIIB) :
a) avec un composé de formule *(R)*-CH₃OCH₂CH(CH₃)OH et une base auxiliaire, facultativement dans un solvant inerte et en présence dudit agent de piégeage ; ou
b) avec un composé de formule *(R)*-CH₃OCH₂CH(CH₃)OZ et une base auxiliaire dans un composé de formule *(R)*-CH₃OCH₂CH(CH₃)OH ou un solvant inerte ou bien à la fois ce composé et ce solvant, en présence dudit agent de piégeage ; ou
c) avec un composé de formule *(R)*-CH₃OCH₂CH(CH₃)OZ et un composé de formule *(R)*-CH₃OCH₂CH(CH₃)OH ou un solvant inerte ou bien à la fois ce dernier composé et ce solvant, en présence dudit agent de piégeage, Z représentant un métal.

15. Procédé suivant l'une quelconque des revendications précédentes, dans lequel un composé de formule (III) est formé en couplant un composé de formule (V) avec un composé de formule (VI) ou un de ses sels.

16. Procédé suivant la revendication 15, dans lequel un composé de formule (VI) ou un de ses sels est formé à partir d'un composé de formule (VII) 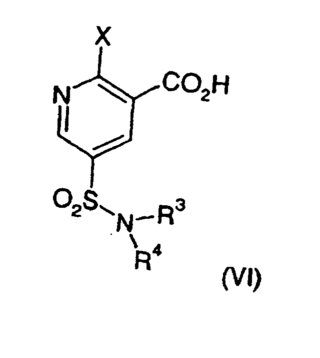 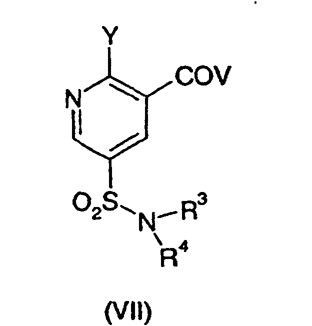
(a) pour un composé de formule (VI) dans laquelle X représente un groupe arylsulfonyloxy, alkylsulfonyloxy en C₁ à C₄, perfluoralkylsulfonyloxy en C₁ à C₄, aryloxy, perfluoralcanoyloxy en C₁ à C₄, alcanoyloxy en C₁ à C₄, (ammonium quaternaire) (alkyle en C₁ à C₄)sulfonyloxy ou halogénosulfonyloxy, par réaction d'un composé de formule (VII) dans laquelle Y et V représentent des groupes OH en présence d'un agent de sulfonylation, d'arylation ou d'acylation approprié de X ;
(b) pour un composé de formule (VI) dans laquelle X représente Cl, par réaction d'un composé de formule (VII) dans laquelle Y représente Cl et V représente P et P représente un groupe protecteur, avec un agent de suppression de protection ;
(c) pour un composé de formule (VI) dans laquelle X représente un diazonium, par réaction d'un composé de formule (VII) dans laquelle Y représente un groupe NH₂, V représente un groupe OH avec l'acide nitreux ;
(d) pour un composé de formule (VI) dans laquelle X représente un groupe diarylsulfonylamino, par réaction d'un composé de formule (VII) dans laquelle Y représente un groupe NH₂ et V représente un groupe OH en présence d'un agent de sulfonylation approprié ;
(e) pour un composé de formule (VI) dans laquelle X représente un groupe OR qui est un groupe alkoxy en C₁ à C₆, par réaction d'un composé de formule (VII) dans laquelle V représente P, P étant un groupe protecteur, et Y représente un groupe alkoxy en C₁ à C₆ primaire ou secondaire, avec un agent de suppression de protection.

17. Procédé pour la préparation de composés de formule (VII) de la manière indiquée dans la revendication 16 à partir de composés de formule (VIII) 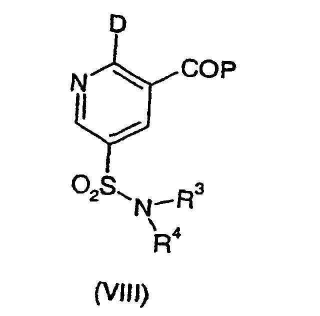 dans laquelle D représente un groupe Cl ou Br et P représente un groupe protecteur,
(a) pour des composés de formule (VII) dans laquelle Y représente un groupe OH et V représente OH, par réaction d'un composé de formule (VIII) avec un agent hydrolysant et éventuellement un agent de suppression de protection supplémentaire lorsque P n'est pas débarrassé de sa protection par l'agent hydrolysant ;
(b) pour des composés de formule (VII) dans laquelle Y représente un groupe NH₂ et V représente un groupe OH, par réaction d'un composé de formule (VIII) avec un agent d'ammoniation pour former un composé intermédiaire de formule (VII) dans laquelle Y représente un groupe NH₂ et V représente un groupe P (un groupe protecteur) et réaction dudit intermédiaire (VII) avec un agent de suppression de protection ; et
(c) pour des composés de formule (VII) dans laquelle Y représente un groupe OR qui est un groupe alkoxy en C₁ à C₆ et V représente un groupe P, par réaction d'un composé de formule (VIII) en présence de ⁻OR.

18. Procédé pour la préparation d'un composé de formule (VIII) suivant la revendication 17, comprenant la réaction de composés de formule (IX) en présence d'une amine de formule NH(R³)(R⁴) dans laquelle R³ et R⁴ répondent aux définitions figurant dans la revendication 1, 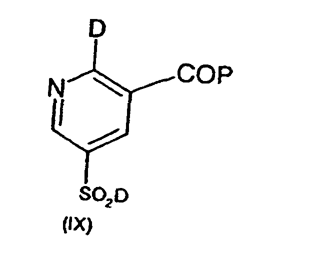 formule dans laquelle D et P répondent aux définitions figurant dans la revendication 17.

19. Procédé pour la préparation d'un composé de formule (IX) suivant la revendication 18, comprenant la réaction d'un composé de formule (X) avec un agent de chloration ou de bromation 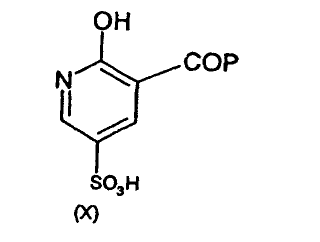 formule dans laquelle P répond à la définition figurant dans la revendication 17.

20. Procédé suivant la revendication 19 oour la préparation d'un composé de formule (X), comprenant la réaction d'un composé de formule (XI) en présence d'un agent qui formera un groupe protecteur (P) sur l'acide carboxylique

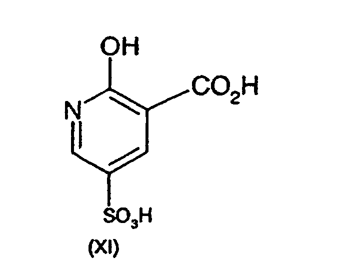

21. Procédé suivant la revendication 20 pour la préparation d'un composé de formule (XI), comprenant la réaction d'acide 2-hydroxynicotinique ou d'un de ses sels en présence de SO₃ dans un solvant.

22. Procédé suivant la revendication 21, dans lequel le SO₃ est présent dans un solvant aprotique, un acide minéral ou un acide carboxylique liquide.

23. Procédé suivant l'une quelconque des revendications 16 à 22, dans lequel les composés de formules (VI), (VII) et (VIII) sont respectivement des composés de formules (VIA), (VIIA) et (VIIIA) 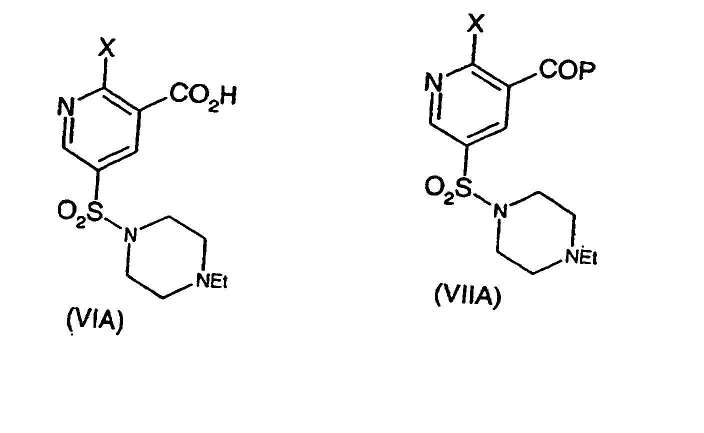 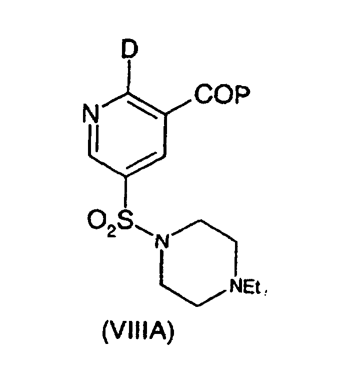 dans lesquelles X représente un groupe alkoxy en C₁ à C₆, D et P répondent aux définitions figurant dans les revendications 16 et 17, et les composés (VIA) et (VIIA) sont formés suivant la revendication 16(e) et la revendication 17(c), respectivement, et le composé (VIIIA) est formé suivant la revendication 18 en faisant réagir le composé (IX) avec de la N-éthylpipérazine ou un de ses sels.

24. Procédé suivant la revendication 23, dans lequel X représente un groupe OEt et le composé (VIA) est formé par réaction du composé (VIIA) avec un agent de suppression de protection, et le composé (VIIA) est formé par réaction du composé (VIIIA) en présence de OEt.

25. Procédé suivant la revendication 23 ou 24, dans lequel le composé (X) est formé en faisant réagir le composé (XI) ou un de ses sels avec de l'éthanol pour former un groupe protecteur OEt.

26. Procédé suivant l'une quelconque des revendications 23 à 25, dans lequel deux ou plus de deux étapes consécutives dans la formation du composé (VIA), (VIIA) ou (VIIIA) sont mises en oeuvre dans du toluène et télescopées ensemble en une seule étape télescopée sans isolement intermédiaire.

27. Procédé suivant l'une quelconque des revendications précédentes pour la préparation d'un composé de formule (I), (IA) ou (IB), comprenant les étapes consistant à partir de l'acide 2-hydroxynicotinique disponible dans le commerce ou d'un de ses sels et à conduire la réaction en présence de SO₃ dans un solvant pour former un composé de formule (XI).

28. Composé (IVA) : 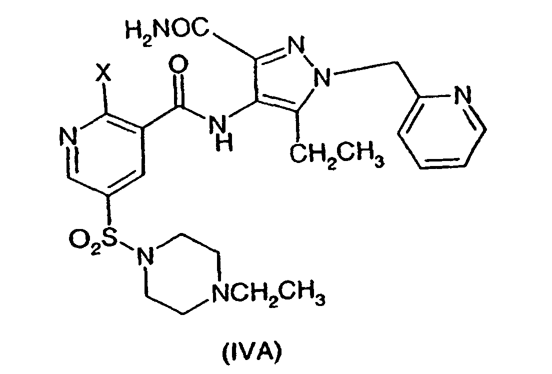 sous réserve que X ne représente pas un groupe méthoxyéthoxy ou éthoxy.

29. Composé (IVB) : 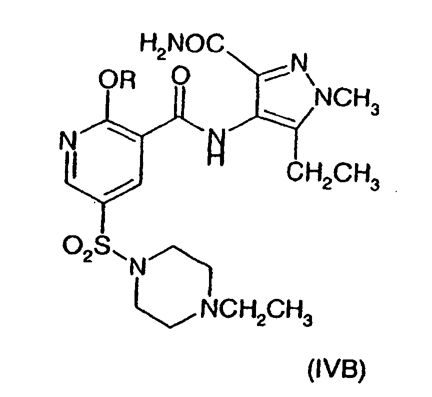 dans lequel R répond à la définition figurant dans la revendication 1, sous réserve que R ne représente pas un groupe éthyle.
